# EUROPEAN PATENT APPLICATION

(11) **EP 4 075 140 A1**
(43) Date of publication of application: **19.10.2022**
(21) Application number: 21167874.3
(22) Date of filing: 12.04.2021
(51) Int. Cl.: G01N 33/58, G01N 33/533, C12Q 1/68, A61K 49/00

(54) **NUCLEIC ACID ORIGAMI PLATFORMS AND THEIR USES**

(71) Applicant: Technische Universität München, 80333 München (DE)
(72) Inventor: DIETZ, Hendrik, 85540 Haar (DE); KICK, Benjamin, 85368 Moosburg (DE); FUNKE, Jonas, 85748 Garching (DE); WAGENBAUER, Klaus, 85748 Garching (DE)
(74) Representative: Engelhard, Markus

(57) **Abstract**

The present invention relates to a nucleic acid nanostructure comprising one or more, preferably at least two, targeting agent(s) and a plurality of dye molecules, preferably at least 10 dye molecules. The present invention further relates to a composition, preferably pharmaceutical composition, comprising a nucleic acid nanostructure. The present invention also relates to a nanostructure or composition for use in medicine, preferably for use in medical imaging, and for use in a method of preventing, treating, and/or diagnosing a disease. Furthermore, the present invention relates to a method of labelling a target in a sample, to the use of a nanostructure as a stain, and to a kit. Moreover, the present invention further relates to a method of preparing a nanostructure. The present invention further relates to a method of identifying a targeting agent combination having a desired property, a desired effect, and/or a desired spatial organization, and to a method of producing a bi- or multispecific targeting agent-comprising molecule. Furthermore, the present invention relates to a method of screening a sample for a target having at least two target molecules.

## Description

### FIELD OF THE INVENTION

The present invention relates to a nucleic acid nanostructure comprising one or more, preferably at least two, targeting agent(s) and a plurality of dye molecules, preferably at least 10 dye molecules. The present invention further relates to a composition, preferably pharmaceutical composition, comprising a nucleic acid nanostructure. The present invention also relates to a nanostructure or composition for use in medicine, preferably for use in medical imaging, and for use in a method of preventing, treating, and/or diagnosing a disease. Furthermore, the present invention relates to a method of labelling a target in a sample, to the use of a nanostructure as a stain, and to a kit. Moreover, the present invention further relates to a method of preparing a nanostructure. The present invention further relates to a method of identifying a targeting agent combination having a desired property, a desired effect, and/or a desired spatial organization, and to a method of producing a bi- or multispecific targeting agent-comprising molecule. Furthermore, the present invention relates to a method of screening a sample for a target having at least two target molecules.

### BACKGROUND OF THE INVENTION

Multispecific antibodies hold great promise as novel drug formats and research tools. For example, the anti-CD3-anti-CD19 bispecific antibody Blinatumomab binds cytotoxic T cells and targets them against CD19-positive tumor cells. Multivalent antibodies have an enormous potential and many drug candidates are currently in preclinical and clinical development, with the focus shifting from bispecific formats towards formats with higher specificities, e.g. trispecific antibodies. Besides their application as therapeutic agents, multispecific antibodies also serve as versatile research tools for investigating cellular mechanisms such as cell-surface receptor assembly.

Monospecific antibodies are readily available against many targets. However, constructing artificial multispecific antibodies is very challenging. Multispecific antibodies are typically created by arranging binding sites on a pre-existing antibody scaffold. Currently, there exist more than 20 different antibody formats that have different properties and valencies. Typically, for providing multispecific antibodies, each new combination of binding sites has to be engineered, produced, and tested separately. This process is therefore laborious, expensive, and slow. Consequently, early-stage research is decelerated, where a large number of candidates has to be tested.

Thus, there is the need for means for efficiently providing bi- or multispecific molecules such as bi- or multispecific antibodies. Furthermore, there is a need to provide means for efficiently targeting targets, such as cells. There is also the need for means that allow to label and/or stain targets, such as cells.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a nucleic acid nanostructure comprising one or more, preferably at least two, targeting agent(s) and a plurality of dye molecules, preferably at least 10 dye molecules, more preferably at least 20 dye molecules, even more preferably at least 30 dye molecules.

In one embodiment, the one or more targeting agent(s) are independently selected from an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a DNA aptamer, a RNA aptamer, a peptide nucleic acid (PNA), a polypeptide, a peptide, a glycoprotein, a peptidomimetic, an anticalin, an Affilin, an Affimer, an Affitin, an Alphabody, a nanobody, DARPin, a receptor ligand, a receptor, a T cell receptor (TCR)-like antibody, a MHC, a pMHC, a receptor domain, and fragments thereof,
preferably independently selected from an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a TCR-like antibody, a MHC, a pMHC, a receptor ligand, and fragments thereof.

In one embodiment, the dye molecules are independently selected from organic fluorophores, e.g. cyanine dyes, Alexa Fluor dyes, Atto dyes, and FITC; quantum dots; fluorescent proteins, e.g. GFP, YFP, RFP, APC, and EGFP; nucleic acid dyes, e.g. Hoechst, DAPI, SYTOX Blue, Chromomycin A3, Mithramycin, YOYO-1, Ethidium Bromide, Acridine Orange, SYTOX Green, TOTO-i, Propidium Iodide, LDS 751, and 7-AAD; Xanthene derivatives, e.g. fluorescein, rhodamine, Oregon green, eosin, and Texas red; Cyanine derivatives, e.g. cyanine, indocarbocyanine, oxacarbocyanine, thiacarbocyanine, and merocyanine; Squaraine derivatives and ring-substituted squaraines, e.g. Seta and Square dyes; Naphthalene derivatives; Coumarin derivatives; Oxadiazole derivatives, e.g. pyridyloxazole, nitrobenzoxadiazole, and benzoxadiazole; Anthracene derivatives, e.g. DRAQ5, DRAQ7, and CyTRAK Orange; Pyrene derivatives, e.g. cascade blue; Oxazine derivatives, e.g. Nile red, Nile blue, cresyl violet, and oxazine 170; Acridine derivatives, e.g. proflavin, acridine orange, and acridine yellow; Arylmethine derivatives, e.g. auramine, crystal violet, and malachite green; Tetrapyrrole derivatives, e.g. porphin, phthalocyanine, and bilirubin; and Dipyrromethene derivatives, e.g. BODIPY, aza-BODIPY.

In one embodiment, said one or more, preferably at least two, targeting agent(s) is/are attached to said nanostructure in a distance of 3 nm to 15 nm, preferably 7 nm to 9 nm from a surface of said nanostructure and/or in a distance of < 5 nm, preferably < 2 nm from a corner of said nanostructure.

In a further aspect, the present invention relates to a composition, preferably pharmaceutical composition, comprising a nucleic acid nanostructure as defined above, optionally further comprising a pharmaceutically acceptable excipient.

In a further aspect, the present invention relates to a nanostructure, as defined above, or a composition, as defined above, for use in medicine, preferably for use in medical imaging.

In a further aspect, the present invention relates to a nanostructure, as defined above, or a composition, as defined above, for use in a method of preventing, treating, and/or diagnosing a disease selected from proliferative diseases, such as cancer, vascular diseases, musculoskeletal disorders, immunological disorders, such as autoimmune diseases, infectious disorders, such as viral diseases e.g. a human immunodeficiency virus (HIV) infection, metabolic disorders, and/or diabetes.

In a further aspect, the present invention relates to a method of labelling a target in a sample, preferably an in vitro and/or ex vivo method of labelling a target in a sample, comprising the following steps:
i) providing a sample,
ii) contacting said sample with the nanostructure as defined above,
iii) optionally, qualitatively or quantitatively determining a labelled target in said sample, wherein, preferably, said determining is performed using flow cytometry, microscopy, e.g. fluorescence microscopy, fluorescence imaging, colorimetry, and/or sequencing, e.g. qPCR.

In a further aspect, the present invention relates to a use of a nanostructure, as defined above, as a stain, preferably as an in vitro and/or ex vivo stain, e.g. as an in vitro and/or ex vivo cell stain.

In a further aspect, the present invention relates to a kit, comprising
i) a nanostructure as defined above;
ii) a nanostructure, comprising at least one first coupling site and at least one second coupling site, wherein said first coupling site is configured to bind at least one dye molecule of a plurality of dye molecules, preferably at least 10 dye molecules, more preferably at least 20 dye molecules, and wherein said second coupling site is configured to bind one or more, preferably at least two, targeting agent(s), a plurality of dye molecules which are configured to bind to said first coupling site, one or more, preferably at least two, targeting agent(s) configured to bind to said second coupling site; and/or
iii) at least one scaffolding strand and a plurality of single-stranded oligonucleotide staple strands, wherein each staple strand is at least partially complementary to at least one scaffolding strand, and wherein each of the staple strands is configured to bind to at least one of the at least one scaffolding strand in at least one place, preferably in two or more distinct places, wherein the at least one scaffolding strand is folded and/or arranged such that the desired nanostructure is formed, further comprising a plurality of dye molecules configured to bind to at least one first coupling site of a scaffolding strand or staple strand, and
   at least one targeting agent configured to bind to at least one second coupling site of a scaffolding strand or staple strand.

In one embodiment, said nanostructure, said targeting agent, and/or said dye molecule are as defined above.

In a further aspect, the present invention relates to a method of preparing a nanostructure as defined above, comprising the steps:
i) providing a nucleic acid nanostructure, a targeting agent, and a plurality of dye molecules,
ii) obtaining a nucleic acid nanostructure comprising said targeting agent and said plurality of dye molecules, preferably by self-assembly of said nanostructure.

In one embodiment, said nanostructure, said targeting agent, and/or said dye molecule are as defined above.

In a further aspect, the present invention relates to a method of identifying a targeting agent combination having a desired property, a desired effect, and/or a desired spatial organization, wherein said method comprises the steps:
a) providing at least one nucleic acid nanostructure, preferably a nucleic acid nanostructure library, wherein said nanostructure comprises a targeting agent combination of at least two targeting agents,
   wherein, optionally, said at least two targeting agents have a defined spatial organization,
b) contacting a sample, preferably a cell, with the nanostructure(s),
c) qualitatively or quantitatively determining a response to said nanostructure in said sample, wherein said response is indicative of the desired property, desired effect, and/or desired spatial organization,
d) identifying the targeting agent combination having the desired property, desired effect, and/or desired spatial organization by identifying a nanostructure comprising the targeting agent combination having the desired property, desired effect, and/or desired spatial organization,
   wherein said desired property is
   1) an affinity or specificity for a target, preferably a receptor or ligand,
   2) a biodistribution profile and/or a pharmacokinetic profile,
      wherein said desired effect is
   3) a cell response via receptor clustering,
   4) a cell and/or receptor activation,
   5) a cell and/or receptor inhibition,
   6) an activation or inhibition of receptor and/or nanostructure internalization, and/or
   7) a clustering of cells, preferably a clustering of cells inducing immune cell activation,
      and/or wherein said desired spatial organization is
   8) an epitope-to-cell-surface distance, e.g. a receptor height, and/or
   9) an epitope-to-epitope distance, e.g. a receptor proximity.

In one embodiment, said nanostructure further comprises at least one dye molecule and/or said nanostructure is a nanostructure as defined above.

In one embodiment, said qualitatively or quantitatively determining a response in step c) is performed using any of fluorescence measurements, e.g. flow cytometry, fluorescence microscopy, or via microplate reader, luminescence measurements, microscopy, a colorimetric measurement, and/or cell-surface marker staining.

In one embodiment, said nanostructure, said targeting agent, said dye molecule, and/or said qualitatively or quantitatively determining are as defined above.

In a further aspect, the present invention relates to a method of producing a bi- or multispecific targeting agent-comprising molecule, preferably a bi- or multispecific antibody or antigen-binding fragment thereof, comprising the steps:
i) performing the method of identifying a targeting agent combination, as defined above, and
ii) producing a bi- or multispecific targeting agent-comprising molecule, preferably a bi- or multispecific antibody or antigen-binding fragment thereof, having the targeting agent combination identified in step d) of the method of identifying a targeting agent combination.

In one embodiment, said targeting agent and/or said method of identifying a targeting agent combination are as defined above.

In a further aspect, the present invention relates to a method of screening a sample for a target having at least two target molecules, comprising the steps:
1) providing a nucleic acid nanostructure comprising a targeting agent combination of at least two targeting agents,
   wherein, optionally, said nanostructure further comprises at least one dye molecule and/or said nanostructure is a nanostructure as defined above,
2) contacting said nanostructure with a sample to be tested for the target having at least two target molecules,
3) qualitatively or quantitatively determining the target in said sample.

In one embodiment, said sample, said nanostructure, said targeting agent, said dye molecule, and/or said qualitatively or quantitatively determining are as defined above.

In a further aspect, the present invention relates to a use of a nanostructure, as defined above, or a composition, as defined above, for the manufacture of a medicament, e.g. a medicament for preventing, treating, and/or diagnosing a disease, preferably selected from proliferative diseases, such as cancer, vascular diseases, musculoskeletal disorders, immunological disorders, such as autoimmune diseases, infectious disorders, such as viral diseases e.g. a human immunodeficiency virus (HIV) infection, metabolic disorders, and/or diabetes.

In a further aspect, the present invention relates to a method of preventing, treating, and/or diagnosing a disease, preferably selected from proliferative diseases, such as cancer, vascular diseases, musculoskeletal disorders, immunological disorders, such as autoimmune diseases, infectious disorders, such as viral diseases e.g. a human immunodeficiency virus (HIV) infection, metabolic disorders, and/or diabetes, comprising administering a nanostructure, as defined above, and/or a composition, as defined above, to a patient in need thereof.

In one embodiment, said administering comprises administering an effective amount of a nanostructure, as defined above, and/or a composition, as defined above, to a patient in need thereof.

In a further aspect, the present invention relates to a nanostructure, preferably as defined above, or a composition, preferably as defined above, for use in a method of identifying a targeting agent combination having a desired property, a desired effect, and/or a desired spatial organization, e.g. as defined above, preferably a desired biodistribution profile and/or a pharmacokinetic profile, wherein said method comprises
a) providing at least one nucleic acid nanostructure, preferably a nucleic acid nanostructure library, wherein said nanostructure comprises a targeting agent combination of at least two targeting agents; wherein, optionally, said nanostructure comprises a DNA barcode; wherein, optionally, said at least two targeting agents have a defined spatial organization;
b) administering the at least one nucleic acid nanostructure to a subject, preferably a human or an animal, e.g. by injection;
c) qualitatively or quantitatively determining a response to said nanostructure in said subject and/or in a sample obtained from said subject, wherein said response is indicative of the desired property, desired effect, and/or desired spatial organization, preferably the desired biodistribution profile and/or pharmacokinetic profile;
d) identifying the targeting agent combination having the desired property, desired effect, and/or desired spatial organization by identifying a nanostructure comprising the targeting agent combination having the desired property, desired effect, and/or desired spatial organization.

In one embodiment, the qualitatively or quantitatively determining a response in step c) comprises analyzing the presence of a nanostructure in a blood sample, body-fluid sample, tissue sample, and/or organ sample obtained from said subject, e.g. by identifying a DNA barcode of said nanostructure.

In one embodiment, said qualitatively or quantitatively determining a response in step c) and/or said identifying the targeting agent combination in step d) comprises sequencing a DNA barcode of said nanostructure.

In one embodiment, said nanostructure, said composition, said method, said desired property, desired effect, desired spatial organization, said targeting agent, said DNA barcode, said sequencing, said administering, said qualitatively or quantitatively determining, and/or said identifying the targeting agent combination are as defined above.

In a further aspect, the present invention relates to a method of identifying a targeting agent combination having a desired property, a desired effect, and/or a desired spatial organization, preferably a desired biodistribution profile and/or a pharmacokinetic profile, wherein said method comprises
a) providing at least one nucleic acid nanostructure, preferably a nucleic acid nanostructure library, wherein said nanostructure comprises a targeting agent combination of at least two targeting agents; wherein, optionally, said nanostructure comprises a DNA barcode; wherein, optionally, said at least two targeting agents have a defined spatial organization;
b) administering the at least one nucleic acid nanostructure to a subject, preferably a human or an animal, e.g. by injection;
c) qualitatively or quantitatively determining a response to said nanostructure in said subject and/or in a sample obtained from said subject, wherein said response is indicative of the desired property, desired effect, and/or desired spatial organization, preferably the desired biodistribution profile and/or pharmacokinetic profile;
d) identifying the targeting agent combination having the desired property, desired effect, and/or desired spatial organization by identifying a nanostructure comprising the targeting agent combination having the desired property, desired effect, and/or desired spatial organization.

In one embodiment, said nanostructure, said method, said desired property, desired effect, desired spatial organization, said targeting agent, said DNA barcode, said administering, said qualitatively or quantitatively determining, and/or said identifying the targeting agent combination are as defined above.

### DETAILED DESCRIPTION

Engineering of existing protein scaffolds to create multivalent and multispecific antibodies is typically time-consuming, laborious, and expensive. The construction and use of the nanostructures of the invention which are high-valency objects is advantageous because such nanostructures rely on established conjugation techniques, e.g. antibody-conjugation techniques, and a modular DNA-origami platform, namely a nucleic acid nanostructure, rather than on complex protein engineering. The nanostructures thus serve as readily available, cost-effective, and modular alternatives to protein scaffolds, especially in settings where a large number of variants need to be tested, e.g. in screening methods. Such nanostructures have a vast array of applications, including the encapsulation and targeted delivery of molecular payloads such as therapeutics, the efficient screening of a large number of antibody combinations from a library of monospecific antibodies, and the construction of multivalent and multispecific devices that can be used as research tools to investigate cellular processes, such as the formation of cytolytic synapses. The modularity of the DNA-origami technology, particularly of the nanostructure of the invention, advantageously allows for quick and efficient adjustment to different indications, assays, and settings.

Particularly, the DNA-origami methodology allows the creation of user-defined biocompatible structures on the nanometer scale. Based on the molecular programmability of nucleic acids e.g. DNA, any 3D shape can be encoded in the sequences of a few nucleic acid strands, e.g. DNA strands. The individual structures are produced in a self-assembly process, with yields close to 100%. Nucleic acid nanostructures, such as DNA-origami objects, are highly addressable and can be modified with several molecules, such as proteins, antibodies, fluorescent dyes, or chemical moieties. The inventors have developed modular nucleic acid nanostructures to quickly and efficiently create multispecific targeting agent combinations, such as antibody-combinations, e.g. from a library of preexisting monospecific antibodies. The means of the invention are advantageous in that such nanostructures can be provided with user-defined valencies, user-defined geometric orientations, and can be used in many established in vitro and in vivo assays. For example, such nanostructures, e.g. multivalent DNA-origami-protein hybrid objects, can be used to efficiently screen for potent antibody or protein combinations. Furthermore, such nanostructures, e.g. multivalent DNA-origami-protein hybrid objects, can be used to create high-affinity and bright cell stains for the use in different cell-staining methods.

The inventors herein provide a method to quickly and efficiently create multispecific antibody-combinations from a library of preexisting monospecific antibodies with known characteristics. The nanostructures, as implemented by the invention, are designed using DNA-origami methods, which enables building discrete nanostructures with structurally well-defined 3D shapes from nucleic acid components, e.g. DNA components. The means of the invention are advantageous in that nucleic acid nanostructures can be efficiently modified with a variety of biomolecules, thus serving as ideal multivalent targeting agent connectors. The means of the invention are also advantageous in that multivalent and multispecific binding effects are provided. Furthermore, the means of the invention are advantageous in that user-defined valencies and specificities can be incorporated into a nanostructure, kit, use, and/or method of the invention.

The inventors have constructed nanostructures, particularly DNA-origami nano-objects, that carry multiple targeting agents such as antibodies. In one embodiment, the targeting agent attachment-position and the attachment strategy (proximal vs. distal) determines the targeting agent's accessibility and thus its binding affinity to target molecules such as cell-surface-bound antigens. By tuning the targeting agent accessibility, the inventors were able to titrate the cell-binding affinity by one order of magnitude. The inventors also explored the multivalent binding of the nanostructures to cell surfaces by incorporating multiple targeting agents, e.g. antibodies, against the same antigen. The binding rate and the binding yield increases, whereas the unbinding rate decreases with the number of mounted targeting agents, indicating multivalent binding. Multivalent binding effects beyond the naturally occurring two binding sites offered by standard IgG molecules may thus be readily exploited. The inventors' modular approach allows to construct bispecific DNA objects which are capable of targeting different targets, such as CD3-positive T cells to CD19-positive B cells and activating the T cells at the target cell. The inventors further improved the T-cell activation by relying on multivalent B-cell targeting. The bispecific DNA-origami objects specifically depleted CD19-positive B cells in cytotoxic T-cell-mediated killing assays, highlighting the applicability and the therapeutic potential of the nanostructures. Finally, the inventors used the nanostructures as a platform to assemble 105 unique multispecific artificial antibodies and tested them in T-cell activation assays. In one embodiment, the nanostructures are modified by attaching further cell-signaling molecules, such as interleukins, or by further increasing the valency. For example, the inventors attached an additional co-stimulatory anti-CD28 antibody to a nanostructure that already carried an anti-CD3 antibody, thereby demonstrating an enhanced T-cell activation over a single anti-CD3 stimulation.

The term "nanostructure", as used herein, relates to a nucleic acid nanostructure, preferably a DNA-origami structure which is composed of one or multiple DNA-origami subunits (entities). In one embodiment, the nanostructure is a DNA origami nanostructure. Readily available nucleic acid nanostructure techniques e.g. DNA origami techniques, which involve comparatively less complex procedures to assemble than standard nanomanufacturing techniques, may be used to manufacture the nanostructure. In one embodiment, the nanostructure is at least partially manufactured using DNA origami techniques. Owing to the self-assembly of DNA origami structures and the readily available software for designing the corresponding scaffolding and staple strands, this is a comparatively less complex manufacturing process compared to standard nanomanufacturing techniques. In one embodiment, a nanostructure is a DNA origami structure. In one embodiment, a nanostructure comprising targeting agents is a nanostructure with said targeting agents attached thereto. For example, said targeting agents can be attached to said nanostructure via a coupling site, such as via a linker, via any surface of said nanostructure, and/or via a core structure of said nanostructure, e.g. via an inner part of said core structure and/or nanostructure. In one embodiment, a nanostructure comprising targeting agents is a DNA origami with said targeting agents attached thereto. In one embodiment, a core structure of a nanostructure comprising targeting agents is a DNA origami, and to such core structure being a DNA origami said targeting agents are attached. In one embodiment, the targeting agents are not part of the core structure, but are attached to the core structure e.g. a DNA origami, optionally via a nucleic acid strand conjugated to the targeting agents. In one embodiment, a nanostructure has a maximum length smaller than 1000 nm, e.g. in the range of from about 10 nm to about 70 nm, preferably about 20 nm to about 50 nm. In one embodiment, a targeting agent is attached to said nanostructure at a distance of 3 nm to 15 nm, preferably 7 nm to 9 nm from a surface of said nanostructure and/or at a distance of < 5 nm, preferably < 2 nm from a corner of said nanostructure. In one embodiment, the term "attached at a distance of 3 nm to 15 nm from a surface of said nanostructure" means that a targeting agent is attached to said nanostructure, preferably via a linker, wherein a distance between the targeting agent and a surface of the nanostructure, e.g. a distance provided by the length of a linker, is in the range of 3 nm to 15 nm. For example, a targeting agent is located 3 nm to 15 nm away from said nanostructure, but is bound to said nanostructure, e.g. via a linker. In one embodiment, the targeting agent is spaced from a surface of the nanostructure in a distance of 3 nm to 15 nm, preferably 7 nm to 9 nm. The advantage of such a distance is that the targeting agent is highly accessible, and improved accessibility over a targeting agent which does not have such distance is provided. In one embodiment, the term "accessible", as used herein, means capable of binding to and/or being bound by a target molecule. In one embodiment, the term a targeting agent being "accessible", in the context of a surface, means that such surface does not sterically hinder the accessibility of such targeting agent. In one embodiment, such a spacing, i.e. distance, between a targeting agent and a surface of a nanostructure has the advantage of reducing steric hindrance. In one embodiment, the targeting agent of the nanostructure is accessible at a distance of from 3 nm to 15 nm, preferably 7 nm to 9 nm, to a surface of said nanostructure. In one embodiment, a targeting agent is attached to the nanostructure at a distance of < 5 nm, preferably < 2 nm from a corner of said nanostructure, e.g. a linker attached to said targeting agent is attached at a distance of < 5 nm, preferably < 2 nm from a corner of said nanostructure. Accordingly, an attachment position, e.g. a position where a targeting agent is directly attached and/or a position where a linker, e.g. a targeting agent-bound linker (i.e. a linker bound to a targeting agent) is attached, has a distance of < 5 nm, preferably < 2 nm from a corner of said nanostructure. In one embodiment, a targeting agent is attached to and/or spaced from said nanostructure at a distance of from 3 nm to 15 nm, preferably 7 nm to 9 nm, with respect to a surface of said nanostructure, and is attached, e.g. via a linker, at a position having a distance of < 5 nm, preferably < 2 nm from a corner of said nanostructure. In one embodiment, a targeting agent is attached at a distal configuration of said nanostructure. In one embodiment, a distal configuration is realized by having the 3' end of the linker's DNA attachment handle protrude from the nanostructure and by attaching the 5' end of the complementary linker DNA to the targeting agent. In one embodiment, a distal configuration is realized by having the 5' end of the linker's DNA attachment handle protrude from the nanostructure and by attaching the 3' end of the complementary linker DNA to the targeting agent. In one embodiment, a linker has at least 16, preferably at least 20, more preferably at least 26 nucleic acid bases. In one embodiment, a targeting agent is spaced from and/or attached to a nanostructure with a linker having at least 16, preferably at least 20, more preferably at least 26 nucleic acids bases.

In one embodiment, a nucleic acid nanostructure, e.g. DNA-origami structure, comprises at least one scaffolding strand and a plurality of single-stranded oligonucleotide staple strands. In one embodiment, a scaffolding strand makes up and/or traverses the main part of a DNA-origami structure and/or a DNA-origami subunit ("entity"). In one embodiment, an entity is a DNA-origami subunit. The term "staple strand", as used in this invention, shall refer to a single-stranded oligonucleotide molecule, which is at least partially complementary to a scaffolding strand. In general, staple strands can be used to introduce, e.g., coupling sites into a DNA-origami structure and/or a DNA-origami subunit ("entity").

The nanostructure, e.g. DNA origami nanostructure, may comprise at least one scaffolding strand, i.e. single-stranded polynucleotide scaffold DNA with a known sequence. The DNA origami structure may further comprise a plurality of single-stranded oligonucleotide staple strands, wherein each staple strand may be at least partially complementary to at least one scaffolding strand. Further, each of the staple strands may be configured to bind to the at least one scaffolding strand, wherein the at least one scaffolding strand may be folded and/or arranged such that the desired nanostructure may be formed. The term "strand", as used herein, relates to a nucleic acid strand, e.g. a DNA and/or RNA strand. A three-dimensional nanostructure may be realized using DNA origami, i.e. by combining scaffolding strands and staple stands to form the required portions and the overall device. Such designs may for example be performed using software such as caDNAno. That is, a nanostructure comprising multiple portions may in some embodiments be made out of one scaffolding strand, whereas in other embodiments portions of a nanostructure may be constructed utilizing a plurality of scaffolding strands.

In one embodiment, e.g. when the nanostructure is at least partially formed by a DNA origami structure and wherein the DNA origami structure comprises at least one scaffolding strand and a plurality of staple strands, the dye and/or targeting agent may be bound to one of the at least one scaffolding strand or a staple strand by means of a linker molecule, wherein the linker molecule may be connected to a DNA strand portion, which is complementary to a portion of the at least one scaffolding strand or to a portion of a staple strand. In one embodiment, the dye and/or targeting agent is/are coupled to the nanostructure via a linker molecule. In one embodiment, a linker molecule is one or more nucleic acid bases, e.g. a single nucleic acid base or a nucleic acid sequence. In one embodiment, a linker molecule of a targeting agent is connected to a DNA strand of said nanostructure. In one embodiment, a coupling site comprises a linker and/or a site configured to bind to a linker.

In one embodiment, the shape of a nanostructure may be any shape, for example, a brick, a rod, a triangular shape, a round shape, a cuboid, i.e. a rectangular shape, a star shape, or any other shape. In one embodiment, the nanostructure is not a globular structure. The nanostructure of this invention can be of any length. In a preferred embodiment, the nanostructure comprises a maximum length, and in a particularly preferred embodiment, the maximum length is smaller than 1000 nm, preferably smaller than 500 nm, such as around 100 nm, or smaller.

In one embodiment, the nanostructure comprises at least one nucleic acid, wherein said nucleic acid may comprise at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N3-P5'-phosphoramidite, a 1',5'-anhydrohexitol nucleic acid (HNA), or a locked nucleic acid (LNA). In one embodiment, the terms "nanostructure", "nano-object", and "nucleic acid nanostructure" are used interchangeably. In one embodiment, when referring to a "core structure" of a nanostructure, the nucleic acid nanostructure without the targeting agents is meant, e.g. a DNA origami comprised by a nucleic acid nanostructure.

In one embodiment, each of the staple strands is configured to bind to at least one of the at least one scaffolding strand in at least one, preferably two or more distinct places. In one embodiment, a nucleic acid nanostructure and/or a DNA origami comprised by a nucleic acid nanostructure has/have a scaffolding strand and one or more staple strands. In one embodiment, a nucleic acid nanostructure and/or a DNA origami comprised by a nucleic acid nanostructure comprise(s) ≤ 100 staple strands. In one embodiment, staple strands have a length of from 20 to 100 nucleic acid bases. In one embodiment, a scaffolding strand has a length of from 100 to 20000 nucleic acid bases, preferably from 120 to 10000 nucleic acid bases, more preferably from 150 to 8064 nucleic acid bases. In one embodiment, a targeting agent is attached and/or bound to the nucleic acid nanostructure via a nucleic acid sequence conjugated to said targeting agent, preferably a nucleic acid sequence that is complementary to a nucleic acid sequence of the nucleic acid nanostructure, e.g. complementary to a scaffolding strand of said nucleic acid nanostructure, and/or is complementary to a nucleic acid handle, preferably DNA handle, attached to and/or incorporated in said nucleic acid nanostructure.

The terms "DNA origami structure" or "DNA-origami objects", as used herein, relate to a nanostructure that comprises DNA as a building material to make nanoscale shapes. Preparing and/or providing a DNA origami involves folding of one or more "scaffolding" DNA strands into a particular shape using a plurality of rationally designed "staple" DNA strands, e.g. by self-assembly. A scaffolding strand is typically longer than a staple strand. The nucleic acid sequences of the staple strands are designed such that the staple strands hybridize to particular portions of the scaffolding strands and, due to the hybridization, a particular shape of the nanostructure is obtained. In one embodiment, the term nucleic acid "handle" relates to a nucleic acid sequence extension capable of binding to a moiety, e.g. a staple strand having extensions capable of binding to a moiety such as a nucleic acid sequence attached to a targeting agent. The term "nucleic acid", as used herein, relates to a nucleotide sequence, such as ribonucleic acid or deoxyribonucleic acid. The nanostructure of the invention is advantageous in that it is stable in any buffer and even in samples such as blood samples. In one embodiment, a nanostructure comprises a DNA barcode, e.g. a DNA barcode which is part of a scaffolding strand and/or staple strand.

The term "patient", as used herein, may relate to a human or an animal. In one embodiment, when referring to a method, the method is an in vivo, ex vivo, in vitro, or in situ method, e.g. an in vitro method. In one embodiment, when referring to a use, the use is an in vivo, ex vivo, in vitro, or in situ use, e.g. an in vitro use. In one embodiment, the nanostructure of the invention is for use in vivo or in vitro, preferably in vivo. The composition, preferably pharmaceutical composition, of the invention shall be formulated to be compatible with its intended route of administration. In a particularly preferred embodiment, examples of routes of administration of the pharmaceutical and/or compound of this invention include intravenous, oral, intranasal, intrathecal, intra-arterial, intradermal, subcutaneous, transdermal (topical), intracerebroventricular, intraparenchymal, intratumoral, transmucosal, rectal, vaginal, bronchial, parenteral administration, and any other clinically/medically accepted method for administration of a pharmaceutical and/or a compound. The term "effective amount", as used herein, relates to an amount sufficient to evoke a desired effect, e.g. a sufficient labeling for in vivo imaging.

In one embodiment, the term "targeting agent" relates to a moiety capable of binding a target and/or target molecule, e.g. a target on a cell surface, preferably specifically binding said target, such as an antigen-binding peptide. In one embodiment, a targeting agent is any of an antibody or an antigen-binding peptide thereof, for example an IgG, a Fab fragment, a single-chain Fab fragment, or a single domain antibody, a DNA and/or RNA aptamer, a peptide, a binding protein, a ligand of a cell-surface receptor (e.g. PD-Li), or a lipid, e.g. any of an antibody or antigen-binding fragment thereof, an antigen-binding peptide, a Fab fragment, a F(ab')₂ fragment, a Fv fragment, a diabody, a single chain Fv fragment, a (scFv)2, a tetrabody, a triabody, a disulfide bond-stabilized Fv (dsFv), a Fc domain, an engineered Fc domain, a DNA aptamer, a RNA aptamer, a peptide nucleic acid (PNA), a polypeptide, a peptide, a glycoprotein, a peptidomimetic, an anticalin, an Affilin, an Affimer, an Affitin, an Alphabody, a nanobody, DARPin, a receptor ligand, a receptor, a T cell receptor (TCR)-like antibody, a major histocompatibility complex (MHC), a peptide-loaded major histocompatibility complex (pMHC), a receptor domain, and fragments thereof. In a preferred embodiment, for example in a method of identifying a targeting agent combination and/or in a method of screening a sample for a target, targeting agent(s) are independently selected from an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a TCR-like antibody, a MHC, a pMHC, a receptor ligand, and fragments thereof. In one embodiment, the targeting agent(s) is/are not an aptamer. In one embodiment, the targeting agent is a protein. In one embodiment, the targeting agent is a cytokine. In one embodiment, the targeting agent is an antigen-binding peptide, such as an antibody, an antibody-like molecule, an antibody mimetic, an antigen binding derivative, or an antigen-binding fragment thereof. In one embodiment, the interaction between a targeting agent and its target is characterized by the specific binding of an antibody-antigen interaction and/or a ligand-receptor interaction. The person skilled in the art is able to identify similar specific interactions, which shall also be within the scope of this invention. As an example, the specific binding of an aptamer to a molecule, or of a receptor ligand to its receptor, may also be used, or vice versa. However, a great variety of interactions may be utilized in such a nanostructure, which may be advantageous compared to the state of the art that relies on specific and limited interactions, e.g. interactions of a receptor and a ligand. Thus, owing to the wide range of interactions available, more versatile and adjustable means, e.g. DNA nanostructure platform, are provided compared to nanostructures disclosed in the prior art. For example, the targeting agent can be an IgG antibody, an IgG antibody fragment, an IgG antibody mimetic, an IgM antibody, an IgM antibody fragment, an IgM antibody mimetic, an IgD antibody, an IgD antibody fragment, an IgD antibody mimetic, an IgA antibody, an IgA antibody fragment, an IgA antibody mimetic, an IgE antibody, an IgE antibody fragment, or an IgE antibody mimetic. In one embodiment, a MHC is a MHC class I or a MHC class II. In one embodiment, a pMHC is a pMHC class I or a pMHC class II. In one embodiment, the targeting agent is not streptavidin or biotin. In one embodiment, the nanostructure does not comprise a i) fluorescent protein and ii) biotin or streptavidin.

The term "specific" or "specifically binding", as used herein, means in accordance with this invention that the targeting agent, e.g. antibody or antigen-binding peptide, is capable of specifically interacting with and/or binding to a specific target or a set of specific targets but does not essentially bind to other molecules, such as antigens. Such binding may be exemplified by the specificity of a lock-and-key-principle. In one embodiment, when referring to the term "binding", such term is to be construed to comprise the term "specific binding". In one embodiment, when referring to "targeting", such term is to be construed to comprise the term "specific targeting".

In one embodiment, the targeting agent reversibly or irreversibly binds to its target. For example, reversibly binding molecules may be an antibody, an antibody fragment, a DNA strand, a biotin molecule, a streptavidin molecule, whereas irreversibly binding molecules may be a maleimide-thiol chemistry molecule or a click chemistry molecule. In one embodiment, when more than one targeting agent is comprised by a nanostructure, the targeting agents may be of the same type or of different types. In one embodiment, when more than one targeting agent is comprised by a nanostructure, the targeting agents may target the same target molecule and/or different target molecules, preferably target different target molecules. In one embodiment, an exemplary targeting agent is any of anti-CD3 epsilon (e.g. Fab), anti-CD28 (e.g. Fab), IL2, anti-CD19 (e.g. Fab), anti-CD123 (e.g. Fab), and anti-CD33 (e.g. Fab), anti-CD137 antibody, anti-CTLA-4 antibody, anti-PD-i antibody, PD-Li, or combinations thereof.

The term "antigen-binding peptide", as used herein, relates to a peptide which specifically binds to an antigen. In one embodiment, an antigen-binding molecule is based on an immunoglobulin, such as a polyclonal or monoclonal antibody, or based on a protein scaffold structure having antigen-binding capacity, such as an anticalin protein, an Affilin, an Affimer, an Affitin, an Alphabody, a nanobody, or a DARPin. In one embodiment, an antigen-binding peptide relates to an antibody or an antibody fragment. In one embodiment, the terms "antibody", "antibody fragment", and "antigen-binding peptide" are used interchangeably. There are several classes of antibodies, particularly human antibodies, such as IgA, IgG, IgM, IgD and IgE, as well as subclasses, such as IgG1, IgG2, IgG3, IgG4, IgA1, IgA2, IgM, IgD, and IgE. Also useful for the invention may be IgGs that are hybrid compositions of the natural human IgG isotypes. In one embodiment, an antigen-binding peptide and/or antibody may comprise a domain of any antibody class, subclass, and/or chain. In one embodiment, the amino acid sequence of an antigen-binding peptide may be modified by protein engineering, e.g. to comprise constant regions from other immunoglobulin classes mediating improved binding properties, e.g. effector function properties, such as immune cell stimulating or immune cell inhibiting functions.

According to the present invention, an antigen-binding peptide may relate to, for example, human antibodies, human-chimeric antibodies, humanized antibodies, and antibody fragments. An antibody recognizes an antigen via the Fab fragment variable region comprising a paratope. Said paratope specifically targets an epitope on an antigen. According to the present invention, an antigen-binding peptide may further relate to a fragment of an antibody, such as a substantially intact antibody, a Fab fragment, a F(ab')2 fragment, a diabody, a single chain Fv fragment, a tetrabody, a triabody, a disulfide bond-stabilized Fv (dsFv), or a heavy chain VHH fragment from camels. However, other forms of an antigen-binding peptide are also envisioned by the present invention. For example, the antigen-binding peptide may be another (non-antibody) receptor protein derived from small and robust non-immunoglobulin scaffolds, such as those equipped with binding functions for example by using methods of combinatorial protein design. Such a non-antibody antigen-binding peptide may be, for example, an Affibody molecule, an Affilin, an Affimer, an Affitin, an Alphabody, an Anticalin, a nanobody, or a DARPin. An antigen-binding peptide may also relate to other antibody-mimetic molecules such as a dual-affinity re-targeting antibody (DART). In one embodiment, an antigen-binding peptide preferably relates to an antibody or an antibody fragment. In one embodiment, an antigen-binding peptide may relate to any antibody-mimetic molecule which mimics the binding properties of an antibody to an antigen but which is structurally distinct from a naturally occurring antibody.

The term "human antibody", as used herein, relates to an antibody which only comprises human sequences, or a fragment thereof. In one embodiment, a human antibody is produced by a display technique, such as by phage display or ribosome display. The term "humanized antibody", as used herein, relates to an immunoglobulin chain or fragment thereof, such as a Fab fragment, a Fab' fragment, a F(ab')2 fragment, a Fv fragment, or another antigen-binding fragment of an antibody, which contain a sequence from non-human immunoglobulin. In one embodiment, a humanized antibody is a human antibody in which certain CDR residues are replaced by CDR residues from a non-human having desired specificity and affinity properties. The term "chimeric-human antibody", as used herein, relates to an artificial antibody which has been designed by genetic engineering or protein engineering by combining sequences of different species, such as mouse and human. In one embodiment, a human-chimeric antibody is a hybrid protein of the antigen-binding domain from a mouse antibody and the constant domain of a human antibody. The term "substantially intact antibody", as used herein, relates to an antibody which is not fragmented, truncated or otherwise shortened, and which has retained its capability of specifically binding to a target such as an antigen.

The term "targeting", as used herein, relates to the capacity of a molecular structure, e.g. a targeting agent, such as an antibody, an antigen-binding peptide, or a surface molecule of a cytotoxic T cell, to bind to a certain structure, such as an antigen, specifically an epitope, by specific interaction. In one embodiment, a molecule on a target, i.e. , target molecule, is targeted by a targeting agent. In one embodiment, the terms "targeting" and "recognizing" are used interchangeably. The term "cross-reacting", as used herein, relates to off-target binding to a structure and/or molecule which is not the target. In one embodiment, the multifunctional nanostructure used in methods of the invention reduce cross-reactions, since targets are only bound if at least two target molecules on said target are bound, and thus the specificity is increased, and off-target binding and side effects are reduced.

In one embodiment, the terms "one or more" and "at least one", in the context of a targeting agent, may relate to one or more molecules of one or more types of targeting agents being present. For example, two targeting agent molecules may be present, wherein the two targeting agent molecules are of the same type or of different types; e.g. of the same type such as two antibodies of the same type targeting the same antigen, or of different types, such as an antibody targeting one target molecule, e.g. antigen, and a receptor targeting a different target molecule, or two antibodies targeting different antigens. Furthermore, additional targeting agents may be present, such as third, fourth, fifth or sixth targeting agents, wherein each of said third, fourth, fifth, or sixth targeting agents may be of the same or of a different type.

The term "plurality of dye molecules", as used herein, relates to a plurality, e.g. at least three, dye molecules. For example, a plurality of dye molecules may relate to at least 3, 4, 5, 6, 7, 8, 9, 10, or more dye molecules. In one embodiment, a plurality of dye molecules is preferably at least 10 dye molecules, more preferably at least 20 dye molecules, even more preferably at least 30 dye molecules, even more preferably at least 40 dye molecules, even more preferably at least 50 dye molecules. In one embodiment, the plurality of dye molecules comprises dye molecules of the same type or of different types. For example, a plurality of dye molecules comprising 10 dye molecules may comprise 5 dye molecules of one type, e.g. an Alexa Fluor dye, and 5 dye molecules of a different type, e.g. GFP. A nanostructure of the invention is advantageous in that a plurality of dye molecules is provided which allows for bright staining of targets. Thus, surprisingly, target molecules which are present in low numbers can be efficiently labelled. Unexpectedly, such nanostructures enable efficient imaging even of target molecules which are characterized by low abundance and/or expression. Imaging, such as fluorescence imaging, is thus greatly enhanced. Furthermore, nanostructures of the invention can be provided with unique dyes or combination of dyes which allows for simultaneous imaging of several target molecules in one sample using more than one nanostructure, wherein each nanostructure has a unique dye or dye combination. The nanostructures of the invention are advantageous in that staining is even possible for targets with low expression levels and/or for targets for which no specific or affine antibody exists. In one embodiment, by using more than one targeting agent and/or making use of avidity, the specificity of a staining/labelling with a nanostructure of the invention can be even further enhanced.

The nanostructures of the invention have at least three advantages over existing antibody conjugates, namely enhanced brightness, increased affinity, and modularity.

### Enhanced brightness

For antigens with low expression levels, i.e. low number of antigens on the cell surface, bright antibody conjugates are rarely available. This is because the number of fluorescent dyes per antibody is limited. Increasing the number of dyes typically impairs the antibody's affinity or its biophysical properties (e.g. solubility) or reduces the fluorescence of the dye. In contrast, the herein presented nanostructure can be modified with a large number of fluorescent dyes (for example >100 dye molecules) without impairing dye brightness or the binding affinity of the antibody. Thus, the nanostructures are extremely bright targeting agent, e.g. antibody, conjugates with which low-expressed antigens can be stained and detected.

### Increased affinity

New antigens or variants of existing antigens are continuously being discovered. For these newly identified antigens or antigen variants there are often no antibodies with sufficiently high affinity available. Instead of increasing the affinity of the antibody through selection methods, which is costly and time-consuming, the nanostructures of the invention use avidity to create targeting agent-nanostructure conjugates with high affinity and high specificity. The avidity is achieved by mounting multiple targeting agents, e.g. weakly binding antibodies, on the same carrier platform, i.e. nanostructure. The approach to use many interactions, such as many weak interactions, to generate a strong interaction, is especially attractive when the bond between the ligands and the cell receptors is inherently weak, for example in pMHC-TCR immune cell screening (tetramer screening).

### Modularity

Because nucleic acids such as DNA can be modified with a wide variety of dyes, e.g. fluorescent molecules, the properties, e.g. fluorescent properties (e.g. excitation wavelengths, emission wavelengths, photostability, and brightness), may be tuned to the user's requirements. In addition, the number and the type of attached targeting agents, e.g. antibodies, and thus the effective affinity of the conjugate can be precisely controlled. Finally, because the approach can make use of pre-existing antibodies, or libraries thereof, new combinations of antibodies and dyes can be quickly assembled with minimal cost or effort.

Accordingly, the nanostructures of the invention allow to quickly generate high-affinity and bright targeting agent conjugates, e.g. antibody or protein conjugates, from pre-existing targeting agents, such as antibodies or proteins. Because of these advantages, the nanostructures are highly advantageous for various application, such as user-configurable dyes in flow cytometry, fluorescence microscopy, and immunostaining or as reagents in biochemical and diagnostic assays. The kit of the invention, the use of the invention, and the methods of the invention benefit from the above mentioned advantages of a nanostructure.

The term "dye molecule", as used herein, relates to any molecule that can be visualized using any imaging technique, e.g. a dye or stain, known to a person skilled in the art, e.g. fluorescent dyes, quantum dots, magnetic particles, gold particles, or color substrate-converting enzymes such as peroxidase or alkaline phosphatase. For example, a dye molecule is selected from organic fluorophores, e.g. Cyanine dyes, Alexa Fluor dyes, Atto dyes, and FITC; Quantum dots; fluorescent proteins, e.g. GFP, YFP, RFP, APC, and EGFP; nucleic acid dyes, e.g. Hoechst, DAPI, SYTOX Blue, Chromomycin A3, Mithramycin, YOYO-1, Ethidium Bromide, Acridine Orange, SYTOX Green, TOTO-i, Propidium Iodide, LDS 751, and 7-AAD; Xanthene derivatives , e.g. fluorescein, rhodamine, Oregon green, eosin, and Texas red; Cyanine derivatives, e.g. cyanine, indocarbocyanine, oxacarbocyanine, thiacarbocyanine, and merocyanine; Squaraine derivatives and ring-substituted squaraines, e.g. Seta and Square dyes; Naphthalene derivatives; Coumarin derivatives; Oxadiazole derivatives, e.g. pyridyloxazole, nitrobenzoxadiazole, and benzoxadiazole; Anthracene derivatives, e.g. DRAQ5, DRAQ7, and CyTRAK Orange; Pyrene derivatives, e.g. cascade blue; Oxazine derivatives, e.g. Nile red, Nile blue, cresyl violet, oxazine 170; Acridine derivatives, e.g. proflavin, acridine orange, acridine yellow; Arylmethine derivatives, e.g. auramine, crystal violet, malachite green; Tetrapyrrole derivatives, e.g. porphin, phthalocyanine, and bilirubin; Dipyrromethene derivatives, e.g. BODIPY, aza-BODIPY. In a preferred embodiment, dye molecules are independently selected from organic fluorophores, e.g. Cyanine dyes, Alexa Fluor dyes, Atto dyes, and FITC, quantum dots, fluorescent proteins, e.g. GFP, YFP, RFP, APC, and EGFP, and nucleic acid dyes, e.g. Hoechst and DAPI. In a particularly preferred embodiment, a dye molecule is a fluorescent dye, for example a non-protein organic fluorophore, e.g. a Cyanine dye, Alexa Fluor dye, Atto dye, and/or FITC. In one embodiment, a "derivative" of a compound is a compound that is derived from the respective compound by a chemical reaction, a compound which is structurally based on the respective compound, and/or is a structural analogue of the compound.

In one embodiment, the term "independently selected", as used herein, means that, if more than one molecule of a kind, e.g. two or more targeting agents or two or more dye molecules are present and/or are to be selected, such two or more molecules may be of the same type or of different types, wherein each of the two or more molecules is selected independently from the other molecule(s). In one embodiment, the term "one or more, preferably at least two", in the context of a nanostructure comprising targeting agent(s), relates to a nanostructure comprising at least one, e.g. one or more than one, such as 2, 3, 4, 5 or more targeting agents. In one embodiment, such more than one targeting agents, e.g. 2 or more targeting agents, are targeting agents of the same type, e.g. antibodies targeting the same antigen, or of a different type, e.g. antibodies targeting different antigens or an antibody and a pMHC. In one embodiment, a nanostructure comprises at least two targeting agents of a different type. In one embodiment, a nanostructure comprising at least two targeting agents has a higher specificity, affinity, and/or avidity than a nanostructure comprising only one targeting agent. The nanostructure of the invention is advantageous in that it can be used for efficient imaging such as medical imaging, e.g. for imaging the interior of a body for clinical analysis and medical intervention, or visual representation of the function of some organs or tissues. Particularly, the nanostructure can be labelled with a plurality of dye molecules for efficient medical imaging, and suitable dyes for any imaging technique can be selected, e.g. for any of X-ray radiography, magnetic resonance imaging, ultrasound, endoscopy, elastography, tactile imaging, thermography, medical photography, tomography, nuclear medicine functional imaging techniques, positron emission tomography (PET), single-photon emission computed tomography (SPECT), electroencephalography (EEG), magnetoencephalography (MEG), electrocardiography (ECG), and/or Magnetic Particle Imaging.

The term "target molecule", as used herein, relates to any molecule of a target, such as a chemical compound, a peptide, and antigen, lipid, and/or any other molecule to be bound by a targeting agent, e.g. a surface peptide, surface protein, surface receptor, surface antigen, and/or surface lipid of a target. In one embodiment, the target molecule is a molecule presented on the surface of a target and/or accessible on the surface of the target. In one embodiment, a target molecule such as an antigen is a cell-surface bound molecule or a sensor-surface bound molecule, such as any of proteins, cluster-of-differentiation molecules, receptors, lipids, ion channels, sugars, or any other cell-surface bound molecule or structure. In one embodiment, a target molecule is an antigen.

The term "target", as used herein, relates to any molecule, structure, material, cell, antigen, and/or surface to be targeted, e.g. to be bound by one or more targeting agent(s). In one embodiment, said target is a cell, molecule, material, and/or surface e.g. a sensor surface, immobilization surface, or any other surface used for an assay. For example, a target can be a sensor, an immobilization surface, a bead, a column material of a chromatography column, a microtiter plate surface, a biotinylated or streptavidin-coated surface, and/or any other surface, material, or molecule used for an assay to immobilize analytes such as cells. For example, a nanostructure can be targeted to a first target to immobilize the nanostructure and a second target binding thereto. Such immobilization can be used for performing assays, selecting and/or sorting cells, or any other method or assay known to a person skilled in the art.

In one embodiment, in the context of a method of the invention, the term "providing a nucleic acid nanostructure" comprises providing an assembled nucleic acid nanostructure and/or providing a building material for a nucleic acid nanostructure, such as a scaffolding strand and one or more staple strands. In one embodiment, a kit of the invention provides a nucleic acid nanostructure. In one embodiment, a method of preparing a nanostructure of the invention comprises a step of allowing self-assembly of a nanostructure and purifying said self-assembled nanostructure, for example step i) of said method, i.e. a step of providing a nucleic acid nanostructure, comprises a step of allowing self-assembly and subsequent purification. In one embodiment, allowing self-assembly comprises mixing at least one scaffolding strand and one or more staple strands, optionally further comprises adjusting the ionic strength, e.g. by adding MgCl₂ to 20 mM, and/or further comprises using a temperature protocol running a sequence of temperatures. In one embodiment, said purifying comprises removing the remaining excess of staple strands, e.g. by a precipitation such as PEG-precipitation, filtration, and/or liquid chromatography. In one embodiment, self-assembly and/or a step of allowing self-assembly comprises a step of denaturation and a step of cooling. In one embodiment, a step of denaturation is performed at a temperature of from 50°C to 80°C, preferably 60°C to 70°C, e.g. about 65°C, for a period of from 1 minute to 45 minutes, preferably 10 to 20 minutes, e.g. about 15 minutes. In one embodiment, a step of cooling is performed at a temperature of from 0°C to 70°C, preferably 20°C to 60°C, e.g., about 50°C to 58°C. In a preferred embodiment, a step of cooling is performed as a gradual cooling, preferably at a temperature from about 58°C to about 50°C with a decrease of 1°C per hour. A person skilled in the art understands that the protocol for self-assembly depends on the design and/or nucleic acid sequence of the nanostructure, and that the protocol can be adjusted in line with known protocols for preparing nanostructures. In one embodiment, a kit comprises a protocol for preparing a nanostructure. In one embodiment, a nanostructure is configured to have a scaffolding sequence that does not comprise a sequence forming secondary structures. In one embodiment, a nucleic acid sequence conjugated to a targeting agent, preferably a nucleic acid sequence conjugated to a targeting agent which is complementary to a sequence of the nanostructure, is configured to consist of a sequence that does not form secondary structures. In one embodiment, the nucleic acid sequences of a nanostructure, e.g. the nucleic acid sequences of a scaffolding strand and staple strands, the nucleic acid sequence of a nucleic acid handle e.g. DNA handle, and/or the nucleic acid conjugated to a targeting agent are configured to not form a secondary structure. In one embodiment, a self-assembled nanostructure has one or more vacancies, i.e. single-stranded nucleic acid sequences that can be bound by complementary nucleic acid sequences conjugated to a targeting agent.

In one embodiment, a method of preparing a nanostructure comprises a step of coupling a targeting agent to the nanostructure. In one embodiment, the terms "coupling", "binding to", "attaching to", and "conjugating" are used interchangeably. In one embodiment, the terms "coupled", "bound", "attached", and "conjugated" are used interchangeably. In one embodiment, the step of coupling a targeting agent to the nanostructure comprises providing one or more targeting agent(s), preferably each targeting agent having a unique nucleic acid sequence attached thereto, which is complementary to a nucleic acid strand of the nanostructure, e.g. a scaffolding strand of the nanostructure or a DNA handle strand attached to the nanostructure. In one embodiment, due to the complementarity of the nucleic acid sequence attached to the targeting agents to a nucleic acid sequence of the nanostructure, e.g. a coupling site, such targeting agents bind to the nanostructure via the complementary nucleic acid strands, preferably automatically bind to the nanostructure via the complementary nucleic acid strands. In one embodiment, when referring to a "unique" nucleic acid sequence, such term means that, if a nanostructure comprises more than one targeting agent, the targeting agents have different nucleic acid sequences attached thereto. Such "unique" nucleic acid sequences are configured to be complementary to a specific sequence and/or to bind to a specific location of the nanostructure. In one embodiment, a nucleic acid sequence conjugated to a first targeting agent is different from a nucleic acid sequence conjugated to a second targeting agent.

In one embodiment, the term "coupling site", as used herein, relates to a site, e.g. a nucleic acid sequence, that is configured to connect a nanostructure with a compound such as a targeting agent and/or a dye molecule, e.g. via a linker and/or a complementary nucleic acid sequence. In one embodiment, a coupling site is part of a nanostructure, a scaffolding strand, a staple strand, and/or a linker attached to a nanostructure, such as a DNA handle. In one embodiment, a coupling site is a nucleic acid strand, e.g. a nucleic acid strand configured to bind to a complementary nucleic acid strand. In one embodiment, a first coupling site is configured to bind at least one dye molecule of a plurality of dye molecules, e.g. directly to a dye molecule or to a dye molecule attached to a linker. In one embodiment, a nanostructure comprises one or more first coupling sites. In one embodiment, a first coupling site may be located at more than one positions at the nanostructure. In one embodiment, a second coupling site is configured to bind one or more, preferably at least two, targeting agent(s), e.g. directly to a targeting agent or to a targeting agent attached to a linker. In one embodiment, a nanostructure comprises one or more second coupling sites. In one embodiment, a second coupling site may be located at more than one positions at the nanostructure.

### A method of labelling a target in a sample

In one embodiment, a method of labelling comprises targeting a target, particularly at least one, preferably at least two target molecules of said target, with at least one, preferably at least two targeting agents, respectively. In one embodiment, a method of labelling comprises labelling said target via a plurality of dye molecules attached to the nanostructure, via a DNA barcode encoded in the nanostructure, and/or via an enzyme attached to the nanostructure. In one embodiment, the step of contacting the sample with the nanostructure comprises contacting the sample with a nanostructure comprising one or more, preferably at least two, targeting agent(s) and a plurality of dye molecules, preferably at least 10 dye molecules, optionally further comprising a DNA barcode and/or an enzyme attached thereto. In one embodiment, when contacting the sample with the nanostructure, the target in said sample is labelled with the nanostructure comprising a plurality of dye molecules and optionally a DNA barcode and/or an enzyme. In one embodiment, the step of qualitatively or quantitatively determining a labelled target in said sample comprises determining a signal obtained from said dye, decoding the DNA barcode, and/or determining a signal obtained from a substrate of said enzyme. For example, determining a signal obtained from the dye or substrate may comprise any technique known to a person skilled in the art, particularly imaging, such as microscopy, e.g. fluorescence microscopy, colorimetry, or flow cytometry. For example, decoding a DNA barcode may comprise sequencing, e.g. using qPCR. In one embodiment, the sample in a method of labelling a target in a sample is any of a tissue sample, e.g. plant tissue, animal tissue, or human tissue, a blood sample, a body fluid sample, e.g. urine sample or saliva sample, an analyte, a cell culture, a cell, e.g. prokaryotic or eukaryotic cell, or any other sample of interest for a person skilled in the art. In one embodiment, when referring to a sample to be used in any method of the invention, such sample may also be used in any other method of the invention. In one embodiment, a method of labelling a target in a sample is a histological or cytological method. In one embodiment, the method of labelling a target in a sample of the invention comprises determining a labelled target in said sample, wherein said determining is performed using flow cytometry, fluorescence microscopy, fluorescence imaging, microscopy, colorimetry, and/or sequencing, e.g. via Q-PCR. In one embodiment, the method of labelling a target in a sample of the invention comprises contacting a sample with the nanostructure of the invention, preferably a nanostructure comprising organic fluorophores, optionally further comprising determining a labelled target in said sample e.g. using flow cytometry or fluorescence microscopy.

In one embodiment, the nanostructure of the invention is used as a stain, e.g. a stain for histology or cytology. In one embodiment, the use of a nanostructure as a stain comprises contacting the nanostructure with a sample.

In one embodiment, any of the methods of the invention comprises the use of a kit of the invention. In one embodiment, the kit of the invention can be used to stain and/or label a sample. In one embodiment, the kit comprises a buffer, preferably a TRIS buffer, a HEPES buffer, a buffer comprising NaCl and/or a buffer comprising MgCl₂. In one embodiment, the kit comprises a solution comprising at least 5 mM MgCl₂. In one embodiment, the kit comprises a chemical and/or solution for coupling a DNA strand to a protein, e.g. antibody, such as TRIS, HEPES, or MgCl₂. In one embodiment, any component of a kit, e.g. a nanostructure, a dye molecule, a targeting agent, a scaffolding strand, and/or a staple strand, is/are present in a kit in the form of a solution or in lyophilized form. For example, some components of a kit may be present in the form of a solution and some of the components of a kit may be present in lyophilized form. In one embodiment, the kit comprises several different targeting agents, e.g. at least 3, 4, 5, or 6 different targeting agents, preferably a library of targeting agents, and/or several different dyes, e.g. at least 3, 4, 5, or 6 different dyes, preferably a library of dyes. In one embodiment, the user of the kit can customize the nanostructure according to his/her needs using his/her preferred targeting agents of the several different targeting agents and/or his/her preferred dyes of the several different dyes. In one embodiment, the user can use a kit comprising several targeting agents and/or several dyes to prepare nanostructures having different combinations of targeting agents and dyes. In one embodiment, the kit advantageously allows for staining more than one target in a sample by providing more than one specific combination of dye(s) and targeting agent(s). In one embodiment, the kit comprises instructions for the assembly and/or maintenance of a nanostructure, e.g. instructions for performing a method of preparing a nanostructure of the invention. In one embodiment, a scaffolding strand and/or at least one staple strand of a plurality of single-stranded oligonucleotide staple strands in a kit of the invention comprises a first coupling site and/or second coupling site. In one embodiment, the kit comprises different targeting agents with distinct linkers attached thereto and/or comprises different dye molecules with distinct linkers attached thereto. In one embodiment, a kit of the invention comprises a dye molecule-modified nucleic acid strand, e.g. a fluorophore-modified DNA strand, and/or a targeting agent-modified nucleic acid strand, e.g. an antibody- and/or protein-modified DNA strand. In one embodiment, the use of a kit of the invention comprises performing a method of preparing a nanostructure of the invention, e.g. to assemble a set of nanostructures, wherein each nanostructure has a specific combination of targeting agent(s) and dye molecules.

### A method of preparing a nanostructure

In one embodiment, the method of preparing a nanostructure comprises a step of mixing the nucleic acid nanostructure with one or more targeting agent(s), e.g. at equimolar ratios or with targeting agent excess, optionally further comprising a step of incubating said mixed nucleic acid nanostructure and targeting agents. In one embodiment, the method of preparing a nanostructure comprises a step of mixing the nucleic acid nanostructure, and optionally targeting agent(s), with a plurality of dye molecules, e.g. at equimolar ratios or with dye molecules excess, optionally further comprising a step of incubating said mixed nucleic acid nanostructure, optionally targeting agents, and said dye molecules. In one embodiment, the method of preparing a nanostructure comprises a step of mixing the nucleic acid nanostructure with one or more targeting agent(s) and a plurality of dye molecules, e.g. at equimolar ratios or with excess of targeting agents and/or dye molecules, optionally further comprising a step of incubating said mixture. In one embodiment, the method of the invention comprises a step of removing excess targeting agents, unbound targeting agents, excess dye molecules, and/or unbound dye molecules after a step of mixing a nucleic acid nanostructure with targeting agents and/or dye molecules, e.g. by a precipitation such as PEG-precipitation, filtration, and/or liquid chromatography. In one embodiment, a step of incubating a nanostructure and targeting agents is performed at a temperature of from 20°C to 50°C, preferably of from 30°C to 40°C, e.g. about 37°C, for a period of from 1 minute to 3 hours, preferably 30 minutes to 1.5 hours, e.g. about 1 hour.

In one embodiment, the method of preparing a nanostructure comprises 1) directly incorporating dye molecules and/or targeting agents into the nanostructure and/or 2) attaching dye molecules and/or targeting agents to the nanostructure using a linker, e.g. adapter strand.
1) Directly incorporating dyes, e.g. fluorophores, or targeting agents may comprise assembly of nanostructures by annealing long DNA strands (scaffold strands) with multiple shorter DNA strands (staple strands). Prior to this self-assembling process, the staple strands (or a subset thereof) may be modified with dyes, e.g. fluorophores (such as non-protein organic fluorophores), or targeting agents. After completion of the self-assembling process, the nanostructure comprises the scaffold strand and the dye-modified staple strands or targeting agent-modified staple strands.
2) Dyes, e.g. fluorophores, or targeting agents are attached using linkers, e.g. adapter strands. The staples in a nanostructure, e.g. DNA-origami nanostructure, may be designed to have parts that are not complementary to the scaffold. The assembled nanostructure thus has protruding DNA strands (i.e. adapter strands and/or DNA handles), which may hybridize to dye-modified DNA strands or targeting agent-modified DNA strands that are added after the nanostructure's assembly. For example, targeting agents are modified with DNA strands. These DNA strands are complementary to protruding adapter strands or to the scaffold strand. Alternatively, staple strands may be omitted from the DNA-origami folding process, thus creating cavities. The targeting-agent modified staples or dye-modified staples may be added after the folding process to bind into the corresponding cavities. In one embodiment, a coupling site is a protruding nucleic acid strand, e.g. an adapter strand and/or DNA handle, is a cavity in a nanostructure, and/or is a single stranded binding site of a nanostructure. In one embodiment, the terms "protruding nucleic acid strand", "adapter strand", "DNA handle", and/or "linker" are used interchangeably.

In one embodiment, in a method of preparing a nanostructure of the invention, providing a targeting agent comprises providing one or more targeting agent-modified DNA strands. In one embodiment, in a method of preparing a nanostructure of the invention, providing a plurality of dye molecules comprises providing one or more dye-modified DNA strands, e.g. fluorophore-modified DNA strands. In one embodiment, the method of preparing a nanostructure of the invention comprises a step of mixing targeting agent-modified DNA strand(s) and dye-modified DNA strand(s) with the nanostructure, preferably in equimolar ratio or with excess of the modified strands. In one embodiment, the method of preparing a nanostructure of the invention comprises a step of removing an excess of targeting agent-modified DNA strands and/or dye-modified DNA strands.

In one embodiment, a method of preparing a nanostructure of the invention comprises the steps:
i.i.) providing a nucleic acid nanostructure comprising at least one first coupling site and at least one second coupling site and/or
   providing at least one scaffolding strand and a plurality of single-stranded oligonucleotide staple strands, wherein each staple strand is at least partially complementary to at least one scaffolding strand, and wherein each of the staple strands is configured to bind to at least one of the at least one scaffolding strand in at least one place, preferably in two or more distinct places, wherein the at least one scaffolding strand is folded and/or arranged such that the desired nanostructure is formed, and wherein the at least one scaffolding strand and/or at least one staple strand of the plurality of single-stranded oligonucleotide staple strands comprises at least one first coupling site, and wherein the at least one scaffolding strand and/or at least one staple strand of the plurality of single-stranded oligonucleotide staple strands comprises at least one second coupling site,
i.2.) providing a targeting agent, preferably a targeting agent-modified DNA strand,
i.3.) providing a plurality of dye molecules, preferably a plurality of dye-modified DNA strands and/or a dye-modified DNA strand comprising a plurality of dye molecules;
ii.i.) mixing said provided nucleic acid nanostructure and/or said at least one scaffolding strand and a plurality of single-stranded oligonucleotide staple strands with said targeting agent and said plurality of dye molecules, preferably in equimolar ratio or in excess of the targeting agent and/or the plurality of dye molecules,
ii.2.) optionally, self-assembly,
ii.3.) optionally, removing an excess of the targeting agent and/or the plurality of dye molecules,
ii.4.) obtaining a nucleic acid nanostructure comprising said targeting agent and said plurality of dye molecules.

In one embodiment, providing a targeting agent comprises providing a targeting agent comprising a linker. In one embodiment, providing a plurality of dye molecules comprises providing a plurality of dye molecules comprising a linker. In one embodiment, a linker attached to at least one dye molecule of a plurality of dye molecules is configured to bind to a first coupling site. In one embodiment, a linker attached to a targeting agent is configured to bind to a second coupling site.

### A method of identifying a targeting agent combination

Bi- and multispecific antibodies and proteins have many applications as research tools and as new drug formats. For example, bispecific antibodies are used as T cell engagers to treat cancers - recruiting the body's immune system to combat cancer. They are designed to bind two target molecules (antigens) simultaneously. However, for a given antigen, many different antibodies can link to the same antigen in different ways and with different strengths. Therefore, when developing bispecific antibodies, an effective combination from a large number of potential combinations must be found. The number of possible combinations increases exponentially with the number of valencies and becomes very large, especially for multispecific formats with more than two binding sites. In order to find the best combination, every single antibody or protein combination has to be designed and produced separately. This process is extremely laborious, expensive, and slow and therefore decelerates research and development, especially for constructs with more than two valencies where a large number of candidates has to be tested and evaluated. The method of identifying a targeting agent combination of the invention overcomes such obstacles and is highly advantageous in that it allows to screen a myriad of targeting agent combinations for a targeting agent combination having a desired feature, e.g. evoking a desired effect. Furthermore, the method of identifying a targeting agent combination of the invention is highly advantageous in that it allows to screen a myriad of targeting agent combination positions, i.e. the spatial organization, which is effective in evoking a desired effect.

In one embodiment, in the context of a method of identifying a targeting agent combination having a desired property, a desired effect, and/or a desired spatial organization, a sample is a cell sample, e.g. eukaryotic or procaryotic cell sample, cell culture sample, tissue sample, a body-fluid sample, and/or blood sample. Other samples known to a person skilled in the art, e.g. vesicle systems or bead systems, may be used in a method of identifying a targeting agent combination of the invention.

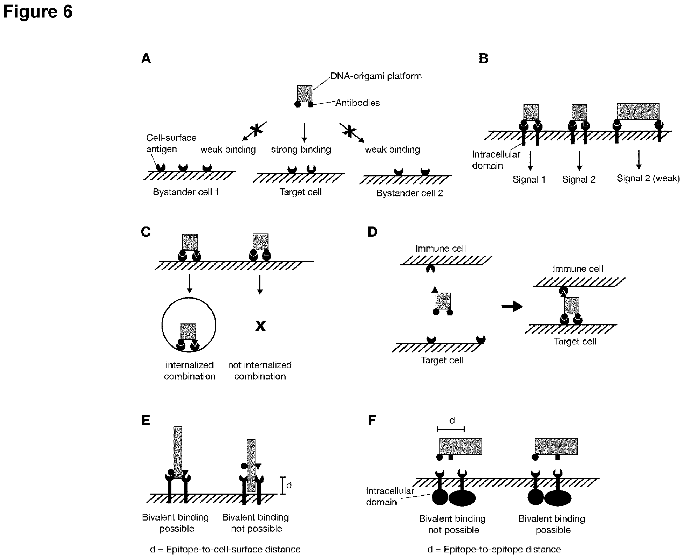

In one embodiment, the terms "desired property", "desired effect", and/or "desired spatial organization" relate to any features which are of interest for a person skilled in the art, e.g. 1) an affinity or specificity for a target, preferably a receptor or ligand, 2) a biodistribution profile and/or pharmacokinetic profile, 3) a cell response, 4) a cell and/or receptor activation, 5) a cell and/or receptor inhibition, 6) an activation or inhibition of receptor and/or nanostructure internalization, and/or 7) a clustering of cells, preferably a clustering of cells inducing immune cell activation, 8) an epitope-to-cell-surface distance, e.g. a receptor height, and/or 9) an epitope-to-epitope distance, e.g. a receptor proximity. In one embodiment, in a method of identifying a targeting agent combination having a desired property, a desired effect, and/or a desired spatial organization, the response to said nanostructure in said sample is qualitatively or quantitatively determined using any of fluorescence measurements, e.g. flow cytometry, fluorescence microscopy, or via microplate reader, luminescence measurements, microscopy, colorimetry, and/or cell-surface marker staining. In one embodiment, a response to said nanostructure in said sample is analyzed and a targeting agent combination having the desired feature is identified by comparing a fluorescence and/or luminescence intensity of a treated sample with an untreated sample, comparing a cell surface marker staining of a treated sample and an untreated sample. In one embodiment, when the desired effect is an activation or inhibition of receptor and/or nanostructure internalization, a fluorescence intensity inside a cell is compared to a fluorescence intensity on the cell surface. In one embodiment, the term "desired feature", as used herein, relates to "desired property", "desired effect", and/or "desired spatial organization". In one embodiment, the desired features analyzed in a method of identifying a targeting agent combination are
1) an affinity or specificity for a target, e.g. measured using fluorescence measurements, e.g. flow cytometry, fluorescence microscopy, or a microplate reader, wherein, for example, the feature is determined by comparing fluorescence intensity of a treated (contacted) sample with an untreated sample (sample not contacted with a nanostructure); for example, a combination of targeting agents, e.g. antibodies or ligands, is identified from a large number of possible combinations such that the identified combination can bind to target cells with increased specificity or affinity (Figure 6A);
2) a biodistribution profile and/or a pharmacokinetic profile, e.g. measured by analyzing samples such as cells, tissue, and/or organs of interest by PCR, qPCR, and/or sequencing, such as samples obtained, preferably minimally or non-invasively obtained, from an animal that was injected with a mixture of nanostructures that carry different targeting-agent combinations and where the targeting-agent combination is encoded in each nanostructure by a DNA barcode. The biodistribution of each targeting-agent combination can be inferred from the occurrences of the corresponding DNA barcodes in each sample, such as an organ;
3) a cell response via receptor clustering, e.g. measured using fluorescence, luminescence measurements, microscopy, or cell-surface marker staining, wherein, for example, the feature is determined by fluorescence or luminescence intensity; for example, a combination of targeting agents, e.g. antibodies or ligands, is identified from a large number of possible combinations such that the identified combination triggers (or inhibits) a desired cellular response (Figure 6B);
4) a cell and/or receptor activation, e.g. measured using fluorescence, luminescence measurements, microscopy, or cell-surface marker staining, wherein, for example, the feature is determined by fluorescence or luminescence intensity;
5) a cell and/or receptor inhibition, e.g. measured using fluorescence, luminescence measurements, microscopy, or cell-surface marker staining, wherein, for example, the feature is determined by fluorescence or luminescence intensity;
6) an activation or inhibition of receptor and/or nanostructure internalization, e.g. measured using fluorescence, luminescence measurements, microscopy, or cell-surface marker staining, wherein, for example, the feature is determined by comparing fluorescence intensity inside a cell with fluorescence intensity on the cell surface; for example, a combination of targeting agents, e.g. antibodies or ligands, is identified from a large number of possible combinations such that the identified combination has high (or low) internalization rates (Figure 6C);
7) a clustering of cells, preferably a clustering of cells inducing immune cell activation, e.g. measured using luminescence measurements, microscopy, or cell-surface marker staining, wherein, for example, the feature is determined by comparing fluorescence intensity of treated cells with untreated cells; for example, a combination of targeting agents, e.g. antibodies or ligands, is identified from a large number of possible combinations such that the identified combination has a high cell-recruiting efficiency, or T-cell activation efficiency, or target-cell-killing efficiency (Figure 6D);
8) an epitope-to-cell-surface distance, e.g. a receptor height, e.g. measured using fluorescence measurements, such as flow cytometry, wherein, preferably, the feature is determined by fluorescence intensity and binding affinity; for example, the distance of the epitope from the surface is inferred from the nanostructure's affinity. A large number of platform variants with varying targeting agent-to-edge distances are generated and tested for their binding affinity. From the binding affinity and the nanostructure's dimensions, the epitope-to surface distance may be inferred (Figure 6E);
9) an epitope-to-epitope distance, e.g. a receptor proximity e.g. measured using fluorescence measurements, such as flow cytometry, wherein, preferably, the feature is determined by fluorescence intensity and binding affinity; for example, the minimal or maximal distance of two epitopes from each other is inferred by measuring the nanostructure's affinity. A large number of platform variants with varying targeting agent-to-targeting agent distances, e.g. antibody-to-antibody distances, are generated and tested for their binding affinity. From the binding affinity and the platform's dimensions, the targeting agent-to-targeting agent distance may be inferred (Figure 6F).

In one embodiment, the nanostructure of the invention and/or a nanostructure provided in any of the methods, kit, and use of the invention comprises a DNA barcode, e.g. a doublestranded or single-stranded DNA sequence. For example, such a DNA barcode is part of a scaffolding strand and/or staple strand. In one embodiment, such DNA barcode can be qualitatively or quantitatively determined using sequencing and/or qPCR. In one embodiment, in the methods or use of the invention, the nanostructure comprises a DNA barcode. In one embodiment, the nanostructure of the invention comprises an enzyme that provides luminescence when contacted with a corresponding substrate. In one embodiment, in the methods, kit, or use of the invention, the nanostructure comprises an enzyme that provides luminescence when contacted with a corresponding substrate. A DNA barcode is advantageous in that a high number, e.g. more than 100 nanostructures, can be analyzed simultaneously, e.g. in a method of identifying a targeting agent combination, and the respective response to a nanostructure can be correlated with the nanostructure using the DNA barcode. For example, in a method of identifying a targeting agent combination having a desired biodistribution profile and/or a pharmacokinetic profile, several different nanostructures, each having a specific targeting agent combination and a specific barcode, can be contacted with a sample, and the nanostructure with targeting agent combination having the desired feature, i.e. showing a desired response in said sample, can be identified by identifying the DNA barcode, e.g. via sequencing and/or PCR.

In one embodiment, providing at least one nucleic acid nanostructure comprising a targeting agent combination comprises a step of modifying at least two targeting agents with DNA strands, preferably DNA strands configured to bind to the nanostructure. In one embodiment, providing a nucleic acid nanostructure library comprises a step of modifying targeting agents with DNA strands to generate a library of targeting agent-DNA conjugates, wherein such targeting agent-DNA conjugates are configured to bind to the nanostructure. In one embodiment, the method of identifying a targeting agent combination comprises a step of mixing and incubating a combination of at least two targeting agent-DNA conjugates with the nucleic acid nanostructure comprising coupling sites configured to be bound by a DNA strand of the targeting agent-DNA conjugate(s), such as protruding adapter strands or single-stranded scaffold regions. In one embodiment, the method of identifying a targeting agent combination optionally comprises a step of removing excess targeting agent-DNA conjugate. In one embodiment, the method comprises stabilizing the nanostructure comprising a targeting agent combination, and/or the nucleic acid nanostructure library comprising targeting agent combinations, against nuclease degradation or low ionic-strength conditions. In one embodiment, providing at least one nucleic acid nanostructure comprising a targeting agent combination comprises providing a library of nucleic acid nanostructures with different combinations of targeting agents. In one embodiment, each nanostructure in such a library comprises a distinct targeting agent combination. In a preferred embodiment of the method of identifying a targeting agent combination, the targeting agents are independently selected from an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a TCR-like antibody, a MHC, a pMHC, a receptor ligand, and fragments thereof. In one embodiment of the method of identifying a targeting agent combination, the targeting agent(s) is/are not an aptamer.

In one embodiment, qualitatively or quantitatively determining a response comprises using a nucleic acid nanostructure comprising a targeting agent combination of at least two targeting agents in a desired assay, e.g. binding-strength assay and/or internalization assay. In one embodiment, the step of qualitatively or quantitatively determining a response comprises determining fluorescence of a sample, such as a cell, in flow cytometry, e.g. when determining affinity or specificity for a target. In one embodiment, the step of qualitatively or quantitatively determining a response comprises comparing internalized fluorescence to fluorescence on a sample surface, e.g. cell surface, e.g. when determining an activation or inhibition of receptor and/or nanostructure internalization. In one embodiment, the step of qualitatively or quantitatively determining a response comprises determining luciferase expression and/or staining of activation markers, such as CD69, e.g. when determining a cell activation, such as a T cell activation. In one embodiment, the method of identifying a targeting agent combination is a high throughput screening method. In one embodiment, the method of identifying a targeting agent combination comprises performing such method using a 96-well plate, wherein one targeting agent combination is tested per well. In one embodiment, a method the invention, e.g. a method of identifying a targeting agent combination, comprises quantitatively determining a nanostructure's DNA barcode in a sample, e.g. a cell sample, a tissue sample, blood sample, and/or body fluid sample. In one embodiment, the method of identifying a targeting agent combination comprises quantitatively measuring the nanostructure's barcode occurrence in organs or blood samples obtained, preferably minimally or non-invasively obtained, from test animals by qPCR or sequencing. In one embodiment, the method of identifying a targeting agent combination is a multiplexed method in which a plurality of targeting agent combinations are tested simultaneously. In one embodiment, such multiplexed method comprises identifying a specific targeting agent combination using a DNA barcode of the respective nanostructure and/or using a specific dye combination, e.g. fluorophore combination. In one embodiment, the method of the invention is advantageous in that it can be performed using multiplexing. In one embodiment, the step of providing at least one nucleic acid nanostructure comprises providing a nanostructure which is labeled with a DNA barcode or with a specific dye and/or dye combination. In one embodiment, after the step of contacting a sample with the nanostructure, and prior to the step of qualitatively or quantitatively determining a response, unbound nanostructures and/or dyes are removed, e.g., by performing a washing step. In one embodiment, the sample and/or the nanostructure is immobilized on a substrate, such as a glass slide, said culture container, and/or well plate. In one embodiment, e.g. after removing unbound nanostructures and/or dyes, a response is qualitatively or quantitatively determined using sequencing, e.g. qPCR, preferably of the DNA barcode, and/or using fluorescent imaging such as flow cytometry for analyzing of the dye and/or dye combination.

In one embodiment, a receptor height and/or receptor proximity is/are identified for effectively designing targeting agent combinations, such as for antibodies or ligands. In one embodiment, in a method of identifying a targeting agent combination having a desired feature, such as 1) an affinity or specificity for a target, 2) a biodistribution profile and/or a pharmacokinetic profile, 3) a cell response via receptor clustering, 4) a cell and/or receptor activation, 5) a cell and/or receptor inhibition, 6) an activation or inhibition of receptor and/or nanostructure internalization, and/or 7) a clustering of cells, when testing more than one nanostructure comprising a targeting agent combination, e.g. when testing a nucleic acid nanostructure library in which each nanostructure has a specific targeting agent combination, the spatial organization of each tested targeting agent combination is similar or the same, so that the qualitative or quantitative determinations of the responses of the tested nanostructures comprising specific targeting agent combinations is comparable.

In one embodiment, a method of identifying a targeting agent combination having a desired property, a desired effect, and/or a desired spatial organization is a screening method for screening a library of nanostructures having various specific targeting agent combinations. In one embodiment, step d) of identifying the targeting agent combination having the desired feature comprises comparing the response to a nanostructure having a specific targeting agent combination with a response to another nanostructure having another specific targeting agent combination, preferably comparing the responses to nanostructures of a nanostructure library, and identifying the nanostructure that has the best response, i.e. the desired feature. In one embodiment, the nanostructure has a DNA barcode and such DNA barcode is used to correlate a determined response to a nanostructure having a specific targeting agent combination. In one embodiment, the nanostructure(s) is/are provided with a defined spatial organization of the targeting agents by using DNA origami technique(s), e.g. the nanostructure is provided with coupling sites for the targeting agents at defined positions of the nanostructure. In one embodiment, a response being "indicative" of the desired feature means that the form, characteristic, severity, and/or extent of the response indicates whether targeting agent combination and/or a nanostructure having a specific targeting agent combination has the desired feature, e.g. the response can be analyzed qualitatively or quantitatively to assess whether a targeting agent combination and/or a nanostructure having a specific targeting agent combination has the desired feature, and optionally the extent of the desired feature. In one embodiment, qualitatively or quantitatively determining a response comprises analyzing the response, e.g. a target binding, and determining whether a response is present or not, optionally further determining to what extent the response is present, e.g. by calculating a value that indicates the extent of the response.

### A method of producing a bi- or multispecific targeting agent-comprising molecule

In one embodiment, a bi- or multispecific targeting agent-comprising molecule relates to a molecule, e.g. an antibody, comprising a targeting agent having more than one specificity and/or comprising more than one targeting agent, e.g. a bispecific antibody comprising two Fab fragments targeting different antigens. Accordingly, such molecule comprises at least two specificities, namely of one or more targeting agents. In one embodiment, the terms "bi- or multispecific" and "bi- or multivalent" are used interchangeably. In one embodiment, a method of producing a bi- or multispecific targeting agent-comprising molecule comprises identifying a targeting agent combination having a desired feature, e.g. a desired property, a desired effect, and/or a desired spatial organization, using a method of identifying a targeting agent combination of the invention. In one embodiment, the information obtained from a method of identifying a targeting agent combination of the invention, namely an information of an efficient targeting agent combination to achieve a desired effect, is used for producing a bi- or multispecific targeting agent-comprising molecule. In one embodiment, a method of producing a bi- or multispecific targeting agent-comprising molecule, e.g. a bi- or multispecific antibody, comprises hybridoma cell culture, protein engineering, synthetic biology, or any other technique known to a person skilled in the art. In one embodiment, the information obtained from a method of identifying a targeting agent combination of the invention, namely an information of an efficient targeting agent combination to achieve a desired effect, preferably an increased target-cell affinity, is used for creating a functional DNA nanostructure with said combination and cytotoxic properties. These cytotoxic properties may be achieved by attaching cytotoxic molecules and/or by attaching immune-cell recruiting antibodies, such as anti-CD3, that lead to immune-cell mediated target-cell killing.

In one embodiment, a method of producing a bi- or multispecific targeting agent-comprising molecule and/or a method of identifying a targeting agent combination are used for personalized medicine. Particularly, a targeting agent combination having a desired feature with respect to a patient is identified using a sample of the patient, e.g. a blood sample, body fluid sample, and/or tissue sample, preferably using a minimally or non-invasively obtained sample from said patient. A nanostructure comprising the identified targeting agent combination and/or a bi- or multispecific targeting agent-comprising molecule which comprises the identified targeting agent combination is/are subsequently administered to said patient. Accordingly, the identified targeting agent combination, a nanostructure comprising the identified targeting agent combination, and/or a bi- or multispecific targeting agent-comprising molecule which comprises the identified targeting agent combination is/are for use in a method of preventing, treating, and/or diagnosing a disease such as cancer, e.g. for use in personalized medicine.

### A method of screening a sample for a target having at least two target molecules

In one embodiment, a method of screening a sample for a target having at least two target molecules, comprises qualitatively or quantitatively determining the target in said sample using any method, such as flow cytometry, fluorescence microscopy, fluorescence imaging, microscopy, colorimetry, and/or sequencing, e.g. via Q-PCR. In one embodiment, a method of screening a sample for a target comprises a step of immobilizing the nanostructure and/or the sample, e.g. on a substrate. In one embodiment, the targeting agents of the nanostructure bind to the respective target molecules on the target. In one embodiment, when the nanostructure is immobilized, such binding of the targeting agents to the target molecules on the target immobilizes the target. In one embodiment, such reversible or irreversible binding and/or immobilization can be used for FACS analyses, e.g. FACS analyses of blood samples, tissue samples, or cell culture samples. In one embodiment, a method of screening a sample for a target having at least two target molecules of the invention comprises a method of labelling a target in a sample of the invention. In one embodiment, a method of labelling a target in a sample of the invention comprises a method of screening a sample for a target having at least two target molecules of the invention. In one embodiment, a method of identifying a targeting agent combination of the invention comprises a method of labelling a target in a sample of the invention and/or a method of screening a sample for a target having at least two target molecules of the invention. In one embodiment, the nanostructure, composition, uses, kit and methods of the invention can be used in any combination. In one embodiment, the method of screening a sample comprises a nanostructure having targeting agents which are pMHC, e.g. a pMHC-tetramer-like screening. In one embodiment, the nanostructure comprises targeting agents which target SARS-CoV2 spike protein(s), and the method of screening is performed to identify cells binding to SARS-CoV2 in a patient sample, e.g. to determine whether a patient is immune to SARS-CoV2.

In one embodiment, a method of screening a sample for a target having at least two target molecules is used for personalized medicine. Particularly, a targeting agent combination is analyzed with respect to whether the corresponding target molecules are present in a patient, using a sample of the patient, e.g. a blood sample, body fluid sample, and/or tissue sample, preferably using a minimally or non-invasively obtained sample from said patient. If such target molecule combination is present in a sample of the patient, it is likely that a treatment targeting the target molecule combination, involving a nanostructure comprising the corresponding targeting agent combination, and/or involving a bi- or multispecific targeting agent-comprising molecule comprising the targeting agent combination is successful in said patient. In one embodiment, the nucleic acid nanostructure in a method of screening a sample is a nanostructure comprising a DNA barcode.

In one embodiment, the terms "a method of the invention" and "the methods of the invention", relate to a method of labelling a target in a sample of the invention, a method of preparing a nanostructure of the invention, a method of identifying a targeting agent combination having a desired property, a desired effect, and/or a desired spatial organization of the invention, a method of producing a bi- or multispecific targeting agent-comprising molecule of the invention, and/or a method of screening a sample for a target having at least two target molecules of the invention. In one embodiment, an embodiment mentioned in the context of a particular method of the invention also relates to other methods of the invention. The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. It will be appreciated, that the use of the singular article "a" or "the" within this document is not meant to limit the scope of the invention expect if specifically stated. That is, generally "a" may also refer to more than one. In other words, "a" can generally be read as "at least one". For example, "a targeting agent" also includes a plurality of targeting agent. In one embodiment, the term "as defined above" relates to "as defined in any of the embodiments above". Whenever method steps are recited in this description and also in the appended claims, it should be noted that the order in which the steps are recited in this text may be accidental. That is, unless otherwise specified or unless clear to the skilled person, the order in which steps are recited may also differ. For example, when the present document states that, e.g., a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) can encompass the situation that step (X) is performed directly before step (Z), but also the situation that step (X) is performed before one or more steps, e.g. (Y1), (Y2), (Y3), followed by step (Z). Corresponding considerations apply when terms like "after" or "before" are used.

### BRIEF DESCRIPTION OF THE FIGURES

The present invention is now further described by reference to the following figures.

All methods mentioned in the figure descriptions below were carried out as described in detail in the examples.

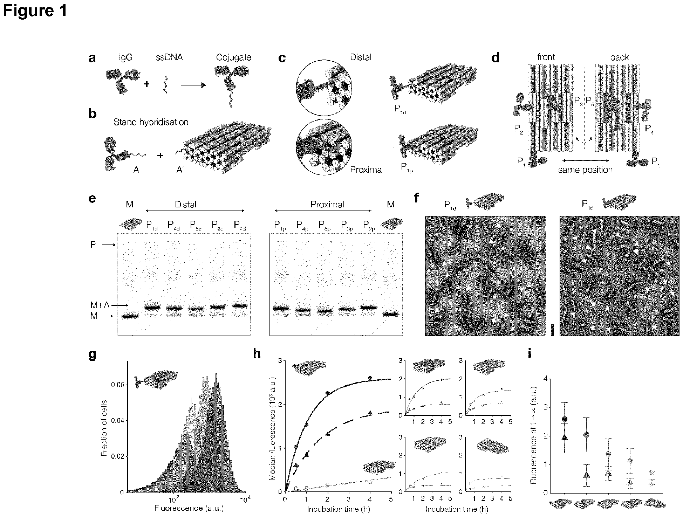

**Figure 1** shows attachment dependent binding of exemplary nanostructures which are antibody DNA-origami constructs. Particularly, **a** to **d** show a schematic representation of a method of preparing a nanostructure, e.g. involving an attachment strategy of full size IgG antibodies (blue) to attachment sites on a nanostructure, e.g. a DNA-origami brick-like object, **a,** Coupling reaction of a nucleic acid, e.g. ssDNA (blue), to a targeting agent, e.g. IgG, thereby obtaining a targeting agent-nucleic acid conjugate. **b,** Binding of such targeting agent-nucleic acid conjugate, e.g. an antibody-DNA conjugate, to a coupling site, e.g. a complementary protruding 3'-end sequence (A'), on the nanostructure. **c,** Sequence-dependent attachment strategy in a proximal (5' end attached to the IgG) or distal (3' end attached to the IgG)) orientation. The targeting agent-nucleic acid conjugate can be attached to a preferred position at the nanostructure, e.g. via a coupling site, to obtain a desired spatial organization. **d,** P1 to P5 indicate the different attachment sites for an anti-CD3 IgG antibody - each position was tested individually. **e,** Fluorescent laser-scanned image of a 2% agarose gel on which the unmodified brick (M) as a reference, (left gel) the brick modified with an anti-CD3 antibody in distal attachment at five different attachment sites, and (right gel) the brick modified with an anti-CD3 antibody in proximal attachment at five different attachment sites were electrophoretically separated. P, M+A, and M highlight the pocket of the gel, the DNA-origami antibody-conjugate band and the monomer band, respectively. The black arrows indicate the individual bands. **f,** Negatively stained TEM micrographs of the nanostructures modified with an anti-CD3 antibody in distal (left) and proximal (right) attachment. Scale bar, 50 nm. White arrowheads indicate the anti-CD3 IgG antibodies attached to the brick-like object, **g,** Flow cytometry analysis of exemplary nanostructures (fluorescent labeled with four Cy5 dyes) binding to CD3 positive T cell leukemia cells (Jurkat). The histograms represent cells only (black), unmodified DNA-origami object (grey), anti-CD3 in proximal (light blue) and distal (blue) attachment at position P1 after 2h of incubation. **h,** Experimentally observed median of fluorescence for different antibody attachment positions (different colors) and configurations (circles: distal; triangles: proximal) determined from histograms as shown in **(g)** as a function of the incubation time. Solid lines represent fits of a simplified binding model to the data. The colored sphere in the schematic icons represent the attachment site of anti-CD3 antibody. **i,** Calculated effective dissociation constants determined from the fits in **(h)** for proximal (triangle) and distal (circle) attachment at different sites as indicated by the icons on the bottom.

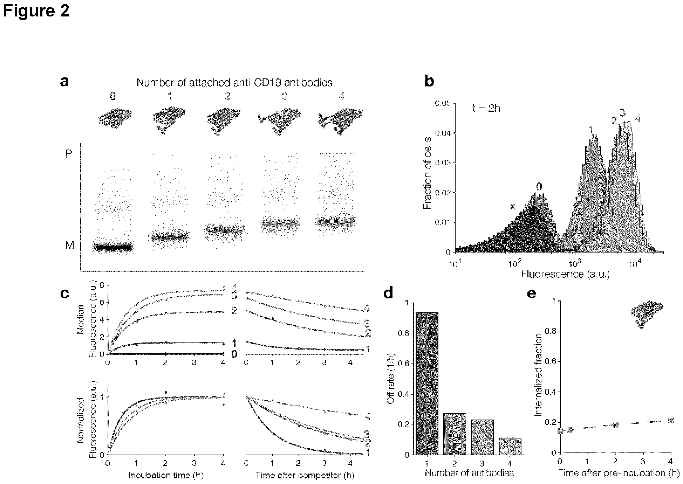

**Figure 2** shows exemplary multivalent nanostructures comprising one or more targeting agents, e.g. multivalent antibody DNA-origami brick constructs. **a,** Fluorescent laser-scanned image of a 2% agarose gel on which the unmodified brick (0) as a reference, and bricks with up to 4 attached anti-CD19 antibodies in distal attachment were electrophoretically separated. Colored icons highlight the position and number of anti-CD19 IgG antibodies. P and M highlight the pocket of the gel and the monomer band, respectively. **b,** Flow cytometry analysis of brick-like structure (fluorescent labeled with 4 Cy5 dyes) binding to CD19 positive precursor B cell leukemia cells (Nalm-6). The histograms represent the cells only (x, purple), the unmodified DNA-origami object (0, dark blue), with one anti-CD19 (1, blue), two anti-CD19 (2m light blue), three anti-CD19 (3, green), and four anti-CD19 (4, orange) in distal attachment. **c,** Left: experimentally observed median fluorescence (two separate measurements circles and triangles) for different numbers of attached anti-CD19 antibodies (different colors and indicated by number of anti-CD19 antibodies) determined from histograms as shown in (**b**) as a function of the incubation time. Solid lines represent fits of a simplified binding model to the data. Right: experimentally observed median fluorescence after a competitor (50-fold excess of free anti-CD19 antibody) is added as a function of the incubation time. Solid lines represent fits of an exponential unbinding model to the data. **d,** Calculated off-rate determined from the fits in (**c**) as a function of the number of attached anti-CD19 antibodies. Inset: normalized fluorescence as a function of the time after the competitor is added. **e,** Left axis: median fluorescence as a function of time after 1h pre-incubation (blue circles). Right axis: internalized fraction of modified DNA-origami objects as a function of time after 1h pre-incubation (blue squares). Solid and dotted lines are fits of an unbinding model (solid) and binding model (dotted) to the data, respectively. Icon indicates the double anti-CD19-modified brick.

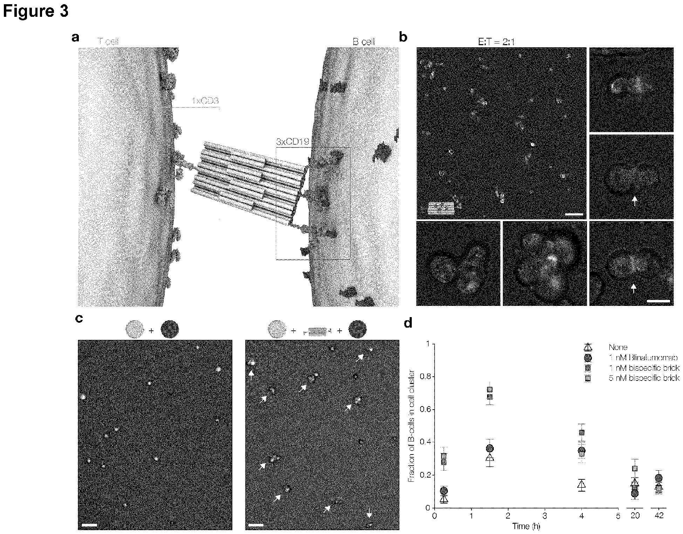

**Figure 3** shows exemplary bispecific nanostructures for methods of the invention, e.g. bispecific DNA-origami constructs for cell-cell recruiting assays. **a,** Schematic representation of the cell-cell recruiting via a bispecific nucleic acid nanostructure. Grey cutouts represent the surface of a T and a B cell with their specific surface antigens CD3 (orange) and CD19 (blue), respectively. The cells are connected via a double antibody-modified DNA brick-like object (grey). The brick is modified with an anti-CD3 (left) and three anti-CD19 (right), respectively. **b,** Fluorescence-microscopy image of a reaction mixture containing T and B cells in a 2 to 1 ratio combined with a fluorescently-labeled bispecific (anti-CD3, anti-CD19) brick. Scale bar, 10 µm. **c,** Fluorescence microscopy image of a reaction mixture containing T and B cells, that were fluorescently stained prior to the experiment, and that were incubated for 1.5h at 37°C in medium without (left) and with a bispecific DNA-origami brick (right). Scale bar, 50 µm. **d,** Fraction of target cells (B cells) recruited to T cells as a function of time. Squares indicate cells incubated with the bispecific brick in different concentrations (orange: 5 nM and red: 1 nM), circles indicate cells incubated with 1 nM of the bispecific antibody Blinatumomab (BiTe - Amgen), and triangles indicate cells incubated without additional recruiting compound. Vertical bars are 95% Wilson-score confidence intervals indicating the statistical error.

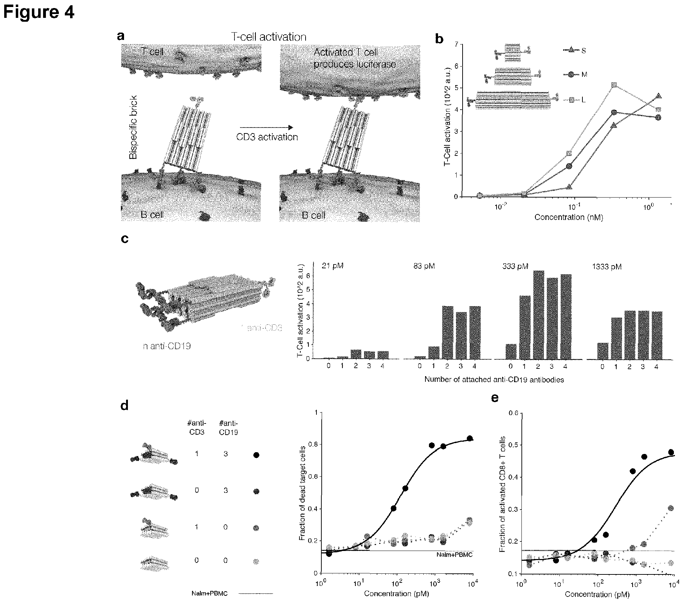

**Figure 4** shows T-cell activation and killing assays for different antibody DNA-origami constructs. **a,** Schematic representation of the T-cell activation assay via a bispecific DNA-origami brick. Grey cutouts represent the surface of a T and a B cell with their specific surface antigens CD3 (orange) and CD19 (blue), respectively. After binding to the CD3 antigen the cells are connected via the antibody-modified brick-like object (grey). The T cells are genetically modified and produce a luciferase upon CD3-receptor activation (yellow color change of the T cell). The luminescence of the T cells is evaluated in (**b**) to (**c**). **b,** T-cell activation via multispecific DNA-origami objects. T-cell activation as a function of the final concentration of the different reactants. Inset indicates the different nanostructures. **c**, T-cell activation via multispecific DNA-origami objects of different lengths. T-cell activation as a function of the final concentration of the different reactants. Inset indicates the length of bricks, modified with anti-CD19 (left) and one anti-CD3 (right) IgG antibodies on the left and the right side of the brick-like objects, respectively. Length of the bricks, S = 25 nm, M = 65 nm, and L = 125 nm, respectively. **d,** T-cell mediated killing of CD19 positive CellTrace-stained B cells (NALM6) after 24h with a nanostructure that carries up to one anti-CD3 Fab fragment and up to three anti-CD19 Fab fragments as indicated in the table on the left. The number of dead target cells was measured using flow cytometry and was calculated by first gating for the Nalm6 target cell based on the CelttTrace fluorescence signal. Dead and alive target cells were identified in the FCS-SSC scatterplot as distinct populations. **e**, Fraction of activated CD8+ T cells obtained from staining the PBMCs with anti-CD69 and anti-CD8 antibodies and gating for them in the respective fluorescence channels.

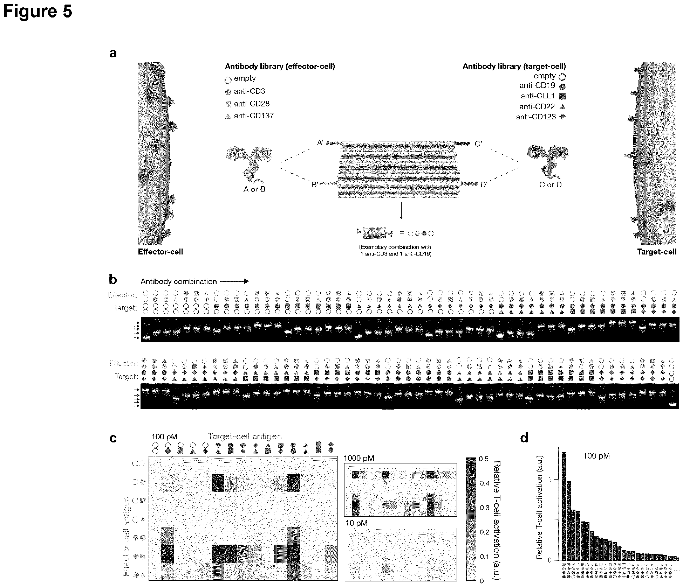

**Figure 5** shows an exemplary method of identifying a targeting agent combination, namely production and functional screening of 105 unique targeting agent combinations, e.g. antibody combinations, using a nucleic acid nanostructure as a platform for attaching various targeting agent combinations. **a,** Schematic of the production of multi-specific brick-antibody variants from four libraries (A, B, C, D) of antibody-DNA conjugates (symbols indicate antibody type, color indicates cell type). Antibodies carry DNA handles with the sequences A, B, C, or D, depending on the library, and the sequences are complementary to coupling sites, such as DNA handles, on the brick object (center). For example, the nucleic acid nanostructure carries four DNA handles. Targeting agent-nanostructure variants, e.g. antibody-brick variants, are produced by mixing the respective targeting agents, e.g. antibodies, from the libraries with the nucleic acid nanostructure, e.g. brick object. Variants are named by their antibody combination (exemplary combination see central bottom). **b,** Laser-scanned image of an agarose gel on which 105 bricks were electrophoresed that were incubated with different antibody combinations (as indicated by the symbols). P, pocket; C, assembled construct. **c,** Relative T-cell activation of the 105 different targeting agent-nanostructure variants for 100 pM, 1000 pM, and 10 pM nanostructure concentration. **c,** Relative T-cell activation of brick variants sorted for maximum activation.
**Figure 6** shows exemplary embodiments of a method of identifying a targeting agent combination of the invention, preferably for screening targeting agent combinations. **(A)** Schematic of the binding of a nucleic acid nanostructure with two attached targeting agents, e.g. antibodies, to a target, e.g. target cell, or an off-target, e.g. bystander cells. The pattern of target molecules on the target, e.g. antigens on the target cell, can only be specifically recognized if the pattern is matched by the corresponding targeting agents, e.g. antibodies, on the DNA-origami platform. Using the herein presented method, a large number of possible targeting agent combinations, e.g. antibody combinations, is efficiently created and screened against target and bystander cells. A method of the invention thus allows determining a targeting agent combination having a desired feature, e.g. the combination that has the highest on-target and lowest off-target affinity. **(B)** Schematic of the nucleic acid nanostructure with attached targeting agents that are bound to cell-surface antigens (e.g. receptors). The cell-surface antigens are brought into close proximity, i.e. clustered, by the nucleic acid nanostructure comprising a targeting agent combination of at least two targeting agents. Clustering of cell surface antigens (or receptors) leads to intracellular signal cascades which may cause cell death, activation, proliferation, activation of gene networks, or more. Using the herein presented method a large number of possible targeting agent combinations, e.g. antibody combinations, are efficiently created and screened for the desired cell-signal response. **(C)** Schematic of nucleic acid nanostructures with attached targeting agents that are bound to cell surface molecules. Cell-surface molecules or combinations thereof are internalized with different rates. In this example, the left targeting agent combination leads to the internalization of the platform whereas the right depicted combination prevents internalization. Using the herein presented method a large number of possible targeting agent combinations, e.g. antibody combinations, may be efficiently created and tested for their internalization rate. **(D)** Schematic of nucleic acid nanostructures with attached targeting agents and two cell types. Two targets, e.g. a target cell (bottom) and an immune cell (top), may be connected by a DNA-origami-antibody platform. Depending on the target-cell and immune cell antibodies, different cell recruiting and immune-cell activation responses may be generated. Using the herein presented method a large number of possible targeting agent combinations (both for the target and immune cell) are efficiently created and tested for their immune-cell recruiting or immune-cell activation or immune-cell-mediated killing efficiency. **(E)** Schematic of nucleic acid nanostructures with attached targeting agents at different distances from the nanostructure's edge. If the targeting agents are attached close to the edge, bivalent binding to the antigens is possible. If the antibodies are attached in the center, bivalent binding to the antigens is not possible. Using the herein presented method a large number of possible targeting agent-to-edge distances, e.g. antibody-to-edge distances, are efficiently created and tested for their binding affinity. The epitope-to-cell-surface distance can thus be inferred from the binding affinity. **(D)** Schematic of nucleic acid nanostructures with attached targeting agents at different antibody distances. If the targeting agents are attached in close proximity, bivalent binding to the antigens is not possible. If the targeting agents are attached in more apart, bivalent binding to the antigens is possible. Using the herein presented method a large number of possible targeting agent-to-targeting agent distances, e.g. antibody-to-antibody distances, are efficiently created and tested for their binding affinity, thus inferring the minimal or maximal antigen-distance.

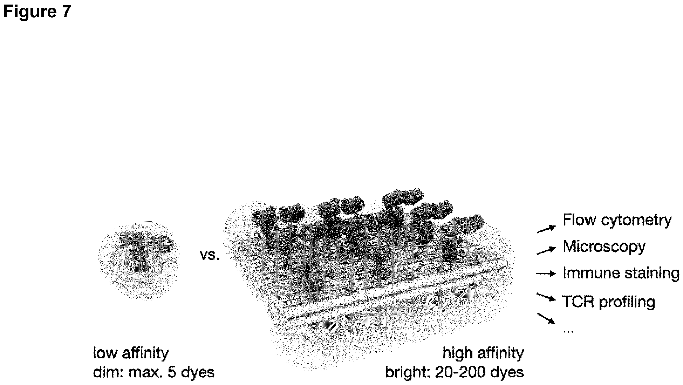

**Figure 7** shows an exemplary nanostructure of the invention comprising at least one targeting agent and a plurality of dye molecules. (Left) targeting agent, e.g. IgG antibody, with dye molecules, e.g. fluorescent dyes (spheres). (Right) Exemplary nucleic acid nanostructure (cylinders), e.g. DNA-origami platform, with nine targeting agents and 60 dye molecules (spheres). The nanostructure of the invention is advantageous in that targets and/or target molecules can be specifically labeled and a higher brightness is achieved for superior imaging, particularly highly effective fluorescent imaging such as fluorescence microscopy.

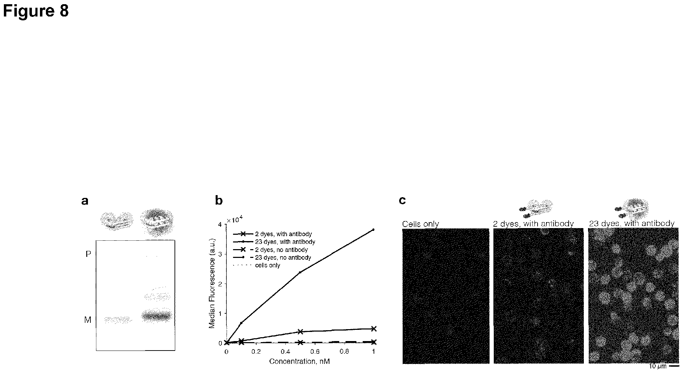

**Figure 8** shows exemplary nanostructures of the invention, e.g. a multivalent and bright cell-targeting DNA-origami-antibody platform. **a,** Fluorescent laser-scanned image of a 2% agarose gel on which the unmodified DNA-origami platform with two Cy5 dyes (left) and 23 Cy5 dyes (right) have been electrophoretically separated. **b,** Median fluorescence cell intensity from flow-cytometry experiments of CD19 positive NALM6 cells that have been incubated with DNA-origami-antibody platforms. Platforms either had two or 23 Cy5 dyes, and carried or did not carry two anti-CD19 Fab fragments. **c,** Fluorescence microscopy image of CD19 positive NALM6 cells that have been incubated with DNA-origami-antibody platforms. Platforms either had two or 23 Cy5 dyes. Images were acquired with same exposure settings and brightness levels and are displayed at same grayscales. The nanostructures of the invention allow for bright staining and/or labeling of targets for highly effective fluorescent imaging.

In the following, reference is made to the examples, which are given to illustrate, not to limit the present invention.

### EXAMPLES

### Example 1: Results

A robust and reliable strategy to rationally position full-size IgG antibodies (e.g., anti-CD19 and anti-CD3) at designed sites on the DNA-origami object is the prerequisite for more complex multi-valent cellular devices. The inventors covalently modified the antibodies with a 25-base NHS-ester activated single-stranded DNA handle (A), where the NHS-ester reacts preferably to lysine residues on the surface of the antibody (Fig. 1a) and purified the DNA-antibody conjugates using ion-exchange chromatography. As an antibody-carrier platform, the inventors used a previously described nanostructure, e.g. brick-like DNA-origami object. The inventors placed single-stranded DNA handles (A') on the surface of the DNA brick at different positions. These protruding DNA handles serve as antibody-attachment sites (Fig. 1b) and may be included by the designer in the folding reaction. Finally, a brick-antibody construct is self-assembled by relying on DNA-DNA hybridization of the protruding strand A' with the complementary antibody-conjugated strand A. By using antibody-modifier strands where the NHS-ester modification is at the 5' or 3' end, the inventors can attach the antibody either in a distal or proximal orientation, respectively (Fig. 1c).

The inventors tested five different antibody positions on the brick to investigate the influence of the position on the cell-binding behaviour. The positions are chosen such that a broad range of 3D accessibility is covered, ranging from the helical interface at the corner (Pi) to the mid of the recess on the bottom side of the brick (P5) (Fig. 1c). For each position P1 to P5, the inventors screened the distal and proximal antibody orientations and analyzed the attachment efficiency of DNA-antibody conjugates to the brick-like structure by agarose gel electrophoresis (Fig. 1e). The attachment of a full-size IgG anti-CD3 antibody (∼180 kDa) to the ∼5 MDa brick in comparison to the unmodified brick object resulted in a noticeable migration difference (Fig. 1e). The inventors could also observe a clear migration difference between the proximal and distal-attachment variants and between the different attachment sites. The differences in migration speeds may be explained by the varying hydrodynamic radius of the antibody-brick objects. Analysis of the bands' cross-sectional profiles showed an overall coupling yield up to 95% (e.g., Pid or P5p). These results were consistent with the TEM data for the P1 position. Single-particle inspection indicated increased flexibility for the distal attachment strategy (Fig. 1d).

The binding affinity between the anti-CD3 antibody and the CD-3 antigen on the cell surface of Jurkat cells, a suspension cell line derived from a patient with acute lymphoblastic leukemia which expresses the CD3 protein complex on the cell surface, was analyzed for ten different fluorescently-labeled brick variants (as analyzed in Fig. 1e). To this end, the inventors performed flow cytometry experiments of brick-cell incubations and calculated the median fluorescence for each variant of the gated Jurkat cells (Fig. 1g). The inventors also corrected the binding curves for variations in antibody-attachment yields as observed in gel electrophoresis. The brick sample without an anti-CD3 antibody showed less binding to the CD3-positive cells compared to bricks with an anti-CD3 antibody, thus indicating specific antibody-antigen mediated binding (Fig. ig, h). For all attachment positions, DNA-origami bricks with an antibody in the distal configuration showed an increased binding affinity compared to bricks with an antibody in the proximal configuration (Fig. 1h). In addition, the binding affinity varies considerably with the attachment site, when comparing only distal or only proximal configurations. For example, the attachment at the corner of the helical interface leads to the highest cell binding affinity, whereas the attachment at the bottom side inside the recess results in the lowest cell binding. These observations may be explained by the differences in antibody accessibilities at the attachment sites (P1 to P5) and by the increased accessibility of the distal configuration compared to the proximal configuration. Thus, the better the antibody's accessibility, the higher the cell-binding affinity. The differences in binding affinities are also consistent with the variations in the migration speed, as a higher accessibility corresponds to a smaller migration speed. By fitting the binding curves with a simplified binding model the inventors were able to evaluate the binding constant for infinite incubation times. This allowed us to quantitatively compare the affinities of the different positions, revealing differences of one order of magnitude.

### Example 2: Multivalent DNA-origami binders

A standard IgG antibody can bind up to two antigens. In order to exploit multi-valency effects with these constructs, the inventors mounted multiple IgG antibodies on the same DNA-origami brick. The inventors' initial findings showed that the highest binding affinity is achieved at the corner of the DNA brick in a distal configuration. The inventors therefore distributed up to four anti-CD19 antibodies to the four corners of the helical interface in a distal configuration (Fig. 2a). Gel electrophoresis of these constructs revealed defined bands that migrate slower than the unmodified brick band and whose migration speeds decrease with the number of attached antibodies (Fig. 2a). The inventors therefore assign these bands to DNA-origami bricks with one to four correctly attached antibodies. The inventors calculated the antibody-attachment yield by cross-sectional profile analysis to 97% for the brick with one antibody and 88% for the brick carrying four anti-CD19 antibodies (Fig. 2a).

Occasionally the inventors also saw lower-molecular weight bands in the gel images below the target lane for brick samples with a designed number of two, three, and four attached antibodies. With the calculated binding efficiency of one antibody, the inventors thus attribute these bands to particles that do not carry all designed antibodies.

The inventors used NALM-6 cells, a suspension cell line derived from a patient with acute lymphoblastic leukemia expressing the antigen CD19, to investigate the binding properties of the multivalent K10-PEG5k-stabilized brick constructs. The inventors performed flow cytometry analysis of incubations with NALM-6 cells and fluorescently tagged DNA bricks with up to four anti-CD19 antibodies. The amount of bound objects increased with the number of attached antibodies. Brick objects without antibodies caused a slight increase in fluorescence signal compared to the sample containing cells only. The brick variant with one antibody showed, however, a much higher fluorescence signal than the unmodified brick object (Fig. 2b). By attaching two anti-CD19 antibodies, the inventors again observed an increase in fluorescence signal. This difference gets less for brick variants with three or four attached antibodies (Fig. 2b). After 4h incubation, the inventors observed a ten-fold increase in binding between the variant with one and the variant with four attached antibodies (Fig. 2c, left). It would be expected that the number of antibodies on the DNA-origami brick will increase the binding rate as the effective antibody concentration is larger for objects with more antibodies. It is therefore difficult to directly deduce if the brick has bound more than one antigen. To test this multivalent binding, the inventors added free unmodified anti-CD19 antibodies in 50-fold excess over the bricks to each sample (Fig. 2c, right). The free antibody should block detached bricks from re-binding. Thus, allowing the inventors to directly observe the brick detachment process. The rate of brick detachment decreased with the number of anti-CD19 antibodies that were mounted on the brick. In order to quantify the brick's off rate, the inventors fitted an exponential decay model to the unbinding data (Fig. 2d). The highest off-rate was found for the brick carrying only one anti-CD19 antibody, whereas bricks with four antibodies had the slowest off-rate and bricks with two or three antibodies had intermediate off rates. The inventors thus conclude that bricks can bind to the cell surface with more than one antibody. Despite the high off-rate for one attached antibody, the inventors still observed a residual fluorescence signal after four hours of brick removal. This signal might point either towards unspecific interactions with the cell surface or toward cellular uptake of the fluorescently-labeled bricks. To investigate these hypotheses, the inventors performed an internalization assay. Briefly, the inventors included a protruding fluorescence single strand on the brick. Upon hybridization of a complementary strand that carries a quencher molecule, the fluorescence is quenched. Since the quencher strand can not penetrate into the cell, the amount of quenched fluorescence corresponds to the fraction of brick objects on the cell surface. After four hours, approximately 20% of the modified bricks have been taken up by the cells (Fig. 2e). The cellular uptake might explain the previously measured residual fluorescent signal.

### Artificial bispecific antibodies

Inspired by T-cell recruiting bispecific antibodies, the inventors designed a DNA-origami brick variant that can carry anti-CD3 antibodies against T cells and anti-CD19 antibodies against B cells (Fig. 3a). The antibodies are attached to opposite sides of the brick at the corners in a distal configuration by using two sets of complementary sequences as DNA handles, for anti-CD3 and for anti-CD19 antibodies, respectively. The inventors used a fluorescently-labeled K10-PEG5k-stabilized brick variant with one anti-CD3 and three anti-CD19 antibodies to test the recruiting of T cells (Jurkat) to B cells (NALM-6) with a 2:1 effector-to-target-cell ratio. Fluorescent microscopy imaging of the cells that were incubated with bispecific brick showed the formation of cell dimers and higher-order cell clusters (Fig. 3b). All cell dimers and clusters showed a fluorescence signal corresponding to the colocalization of fluorescently labeled DNA bricks. The fluorescent signal was mainly located at the interfaces between two or more connected cells (Fig. 3b), supporting a brick induced cell-cell recruiting process.

In order to understand the recruiting process in more detail and to distinguish between the target and effector cells, the inventors stained the two cell types (NALM-6 cells and Jurkat cells) with different fluorescent cell stains and incubated them with non-fluorescent brick variants (Fig. 3c). The inventors used the brick with one anti-CD3 and three anti-CD19 antibodies to favor the binding to the CD19 positive target cells. As references, the inventors used the commercially available anti-CD3-anti-CD19 bispecific antibody Blinatumomab and a sample without additional recruiting agent. At different time points, the inventors collected fluorescence microscopy images of the incubations and counted the number of target cells (NALM-6 cells) that were in a cluster with at least one effector cell (Jurkat cells) (Fig. 3 c and d). For all samples, the fraction of target cells in a cluster is increasing over time, reaching a maximum at 1.5 h, and then decreasing to random cell-cell clustering at 20 h. However, samples with a bispecific brick had an increased fraction of target cells in cell clusters compared with the sample without bricks (70% vs. 30%, respectively). Compared to the bispecific T-cell engager Blinatumomab, the artificial T-cell engagers show a faster clustering effect in the first 4 h after incubation. The inventors attribute this behaviour to a stronger binding affinity for the multispecific bricks in comparison to the BiTe construct. The inventors tested two brick concentrations (1 nM and 5 nM), but observed no difference in the clustering efficiency.

### Example 3: T-cell recruiting and activation using multispecific DNA-origami T-cell engagers

The inventors' bispecific constructs are capable of recruiting T cells to CD19 positive B cells. Next, the inventors wanted to investigate if their constructs are also capable of T-cell activation upon recruitment. To this end, the inventors used genetically modified T cells that express a luciferase upon activation. The degree of T-cell activation can be read out by measuring the luminescence after addition of the luciferase's substrate. The anti-CD3 IgG antibodies that the inventors used can bind to the epsilon chain of the CD3 receptor and should therefore activate T cells (Fig. 4a).

The inventors first tested if the size of the K10-PEG5k-stabilized DNA-origami brick influences the T-cell activation. To this end the inventors constructed three different variants of the brick-like object. The three objects have the same cross-section of 48 dsDNA helices in a honeycomb geometry, but vary in length. The shortest brick is 25 nm in length (S-brick), an intermediate variant is 60 nm long (M-brick), and the longest variant has a length of 125 nm (L-brick) (Fig. 4b). The inventors mounted two anti-CD19 and one anti-CD3 antibodies to these bricks. Activation assays after 6h-incubation yielded an activation signal at brick concentrations of 100 pM and higher. The T-cell activation for the three constructs is similar with a slightly higher activation for the longer constructs (L and M) at low brick concentration. Overall, the inventors do not observe a strong length-dependent for the distances the inventors tested. The inventors therefore proceeded with the 60-nm-long brick variant. As described above, the cell-binding affinity of the antibody-brick constructs may be tuned with the number of attached antibodies. The inventors hypothesized that it may be beneficial to increase the affinity to the target cell (CD19-positive B cells), because T cells are typically present in large excess over target cells (typical effector to target cell ratios are for example 5:1 or 10:1). The inventors therefore compared the T-cell-activation efficiency of brick objects with varying CD19 valencies. Bricks without antibodies or with one anti-CD19 antibody did not cause T-cell activation, as expected (Fig. 4c). A brick with one distal-attached anti-CD3 antibody activated T cells at and above 80 pM brick concentration. However, the T-cell activation increased 4-fold at 300 pM brick concentration once the brick carried both an anti-CD3 and an anti-CD19 antibody. The T-cell activation could be further enhanced by increasing the number of target cell antibodies, enabling T-cell activation at 20 pM brick concentration. Using more than two B-cell targeting anti-CD19 antibodies did not improve the resulting T-cell activation.

The mechanism of action of the bispecific antibody Blinatumomab, relies on recruiting and activating cytotoxic T cells (CD8+ T cells) at the target cell. The inventors therefore tested if the bispecific K10-PEG5k-stabilized DNA-origami constructs are able to induce T-cell mediated killing of target cells (NALM-6 cells). The inventors prepared a bispecific brick variant with three anti-CD19 Fab fragments and one anti-CD3 Fab fragment. As controls, the inventors also created monospecific variant (only one anti-CD3 and only three anti-CD19) and a brick without antibodies. The target cells were fluorescently stained to discern them in flow cytometry experiments from the peripheral blood mononuclear cells. After a 24h incubation at 37°C in complete cell-culture medium, close to all target cells were killed in the incubations with the bispecific brick variant (Fig. 4d). In comparison, the monospecific bricks and the controls showed no target-cell killing up to inM and only slight increased cell death at 10 nM compared to the cells-only control. In addition, the inventors used anti-CD8 antibodies to detect the CD8+ T cells and used anti-CD69 to quantify the level of T-cell activation (Fig. 1e). Again, the bispecific brick variant showed strong T-cell activation whereas the T-cell activation for the monospecific variants was only increased at 10 nM for the anti-CD3-only variant. These results indicate targeted T-cell mediated killing of the B-cell population caused by the bispecific DNA-origami brick.

### DNA-brick platform for the production and screening of multi-specific antibodies

In order to demonstrate the capabilities of the DNA-origami-brick-antibody platform approach, the inventors produced 105 unique multi-specific artificial antibodies and tested their T-cell activation capabilities. The artificial antibodies bind different antigens and combinations thereof on both the effector and target cell. The inventors used a DNA brick with four distinct binding sites. Each binding site carries a DNA handle with a unique sequence (Fig. 5a). For each binding site on the DNA brick, the inventors generated a library of antibody-DNA conjugates that carry the complementary DNA handle. A unique multi-specific antibody-DNA variant is thus produced by mixing the DNA brick with corresponding antibodies. This approach allowed the inventors to generate 105 unique multi-specific antibody-brick variants. For the effector T cell, the inventors chose to test seven combinations that were selected from anti-CD3, anti-CD28, anti-CD137, and no antibodies. For the target B-cell, the inventors chose to test 15 combinations that were selected from anti-CD19, anti-CD22, anti-CLLi, anti-CD123, and no antibodies. The inventors analyzed the 105 different combinations using gel electrophoresis (Fig. 5b). As before, upon binding of an antibody to brick object, a change in migration distance was observed. The yield for a fully assembled multivariant brick varied slightly but approached 100%. Finally, the inventors tested the 105 different variants in T-cell activation assays with NALM6 B-cells and genetically modified T-cells that express luciferase upon activation. Because the inventors were interested in the activation that is caused by a recruited B cell to the T cell, the inventors subtracted the fluorescence of the variants that only carried antibodies against T cells to obtain the relative T-cell activation (Fig. 5c). T cells were only activated when an anti-CD3 antibody was present. Co-stimulation was also observed for variants with an additional anti-CD3, anti-CD137, or anti-CD28 antibody. In addition, the B-cell antibody against the CD19 antigen showed the strongest relative activation. This approach allowed the inventors to rank all antibody combinations by their ability to activate T-cells (Fig. 5d). The strongest activation was achieved by a combination of anti-CD3, anti-CD28 and two anti-CD19 antibodies or one anti-CD19 antibody and one anti-CD22 antibody.

### REFERENCES

The features of the present invention disclosed in the specification, the claims, and/or in the accompanying figures may, both separately and in any combination thereof, be material for realizing the invention in various forms thereof.

## Claims

1. A nucleic acid nanostructure comprising one or more, preferably at least two, targeting agent(s) and a plurality of dye molecules, preferably at least 10 dye molecules, more preferably at least 20 dye molecules, even more preferably at least 30 dye molecules.

2. The nucleic acid nanostructure according to claim 1, wherein the one or more targeting agent(s) are independently selected from an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a DNA aptamer, a RNA aptamer, a peptide nucleic acid (PNA), a polypeptide, a peptide, a glycoprotein, a peptidomimetic, an anticalin, an Affilin, an Affimer, an Affitin, an Alphabody, a nanobody, DARPin, a receptor ligand, a receptor, a T cell receptor (TCR)-like antibody, a MHC, a pMHC, a receptor domain, and fragments thereof,
preferably independently selected from an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a TCR-like antibody, a MHC, a pMHC, a receptor ligand, and fragments thereof.

3. The nucleic acid nanostructure according to claim 1 or 2, wherein the dye molecules are independently selected from organic fluorophores, e.g. cyanine dyes, Alexa Fluor dyes, Atto dyes, and FITC; quantum dots; fluorescent proteins, e.g. GFP, YFP, RFP, APC, and EGFP; nucleic acid dyes, e.g. Hoechst, DAPI, SYTOX Blue, Chromomycin A3, Mithramycin, YOYO-1, Ethidium Bromide, Acridine Orange, SYTOX Green, TOTO-1, Propidium Iodide, LDS 751, and 7-AAD; Xanthene derivatives, e.g. fluorescein, rhodamine, Oregon green, eosin, and Texas red; Cyanine derivatives, e.g. cyanine, indocarbocyanine, oxacarbocyanine, thiacarbocyanine, and merocyanine; Squaraine derivatives and ring-substituted squaraines, e.g. Seta and Square dyes; Naphthalene derivatives; Coumarin derivatives; Oxadiazole derivatives, e.g. pyridyloxazole, nitrobenzoxadiazole, and benzoxadiazole; Anthracene derivatives, e.g. DRAQ5, DRAQ7, and CyTRAK Orange; Pyrene derivatives, e.g. cascade blue; Oxazine derivatives, e.g. Nile red, Nile blue, cresyl violet, and oxazine 170; Acridine derivatives, e.g. proflavin, acridine orange, and acridine yellow; Arylmethine derivatives, e.g. auramine, crystal violet, and malachite green; Tetrapyrrole derivatives, e.g. porphin, phthalocyanine, and bilirubin; and Dipyrromethene derivatives, e.g. BODIPY, aza-BODIPY.

4. The nanostructure according to any one of the foregoing claims, wherein said one or more, preferably at least two, targeting agent(s) is/are attached to said nanostructure at a distance of 3 nm to 15 nm, preferably 7 nm to 9 nm from a surface of said nanostructure and/or at a distance of < 5 nm, preferably < 2 nm from a corner of said nanostructure, e.g. via a linker.

5. A composition, preferably pharmaceutical composition, comprising a nucleic acid nanostructure of any one of claims 1-4, optionally further comprising a pharmaceutically acceptable excipient.

6. The nanostructure of any one of claims 1-4 or the composition of claim 5 for use in medicine, preferably for use in medical imaging.

7. The nanostructure of any one of claims 1-4 or the composition of claim 5 for use in a method of preventing, treating, and/or diagnosing a disease selected from proliferative diseases, such as cancer, vascular diseases, musculoskeletal disorders, immunological disorders, such as autoimmune diseases, infectious disorders, such as viral diseases e.g. a human immunodeficiency virus (HIV) infection, metabolic disorders, and/or diabetes.

8. A method of labelling a target in a sample, preferably an in vitro and/or ex vivo method of labelling a target in a sample, comprising the following steps:
i) providing a sample,
ii) contacting said sample with the nanostructure, as defined in any one of claims 1-4,
iii) optionally, qualitatively or quantitatively determining a labelled target in said sample, wherein, preferably, said determining is performed using flow cytometry, microscopy, e.g. fluorescence microscopy, fluorescence imaging, colorimetry, and/or sequencing, e.g. qPCR.

9. Use of a nanostructure of any one of claims 1-4 as a stain, preferably as an in vitro and/or ex vivo stain, e.g. as an in vitro and/or ex vivo cell stain.

10. Kit, comprising
i) a nanostructure as defined in any one of claims 1-4;
ii) a nanostructure, comprising at least one first coupling site and at least one second coupling site, wherein said first coupling site is configured to bind at least one dye molecule of a plurality of dye molecules, preferably at least 10 dye molecules, more preferably at least 20 dye molecules, and wherein said second coupling site is configured to bind one or more, preferably at least two, targeting agent(s),
a plurality of dye molecules which are configured to bind to said first coupling site, one or more, preferably at least two, targeting agent(s) configured to bind to said second coupling site; and/or
iii) at least one scaffolding strand and a plurality of single-stranded oligonucleotide staple strands, wherein each staple strand is at least partially complementary to at least one scaffolding strand, and wherein each of the staple strands is configured to bind to at least one of the at least one scaffolding strand in at least one place, preferably in two or more distinct places, wherein the at least one scaffolding strand is folded and/or arranged such that the desired nanostructure is formed, further comprising a plurality of dye molecules configured to bind to at least one first coupling site of a scaffolding strand or staple strand, and
at least one targeting agent configured to bind to at least one second coupling site of a scaffolding strand or staple strand.

11. A method of preparing a nanostructure of any one of claims 1-4, comprising the steps:
i) providing a nucleic acid nanostructure, a targeting agent, and a plurality of dye molecules,
ii) obtaining a nucleic acid nanostructure comprising said targeting agent and said plurality of dye molecules, preferably by self-assembly of said nanostructure.

12. A method of identifying a targeting agent combination having a desired property, a desired effect, and/or a desired spatial organization, wherein said method comprises the steps:
a) providing at least one nucleic acid nanostructure, preferably a nucleic acid nanostructure library, wherein said nanostructure comprises a targeting agent combination of at least two targeting agents,
wherein, optionally, said at least two targeting agents have a defined spatial organization,
b) contacting a sample, preferably a cell, with the nanostructure(s),
c) qualitatively or quantitatively determining a response to said nanostructure in said sample, wherein said response is indicative of the desired property, desired effect, and/or desired spatial organization,
d) identifying the targeting agent combination having the desired property, desired effect, and/or desired spatial organization by identifying a nanostructure comprising the targeting agent combination having the desired property, desired effect, and/or desired spatial organization,
wherein said desired property is
1) an affinity or specificity for a target, preferably a receptor or ligand,
2) a biodistribution profile and/or a pharmacokinetic profile,
wherein said desired effect is
3) a cell response via receptor clustering,
4) a cell and/or receptor activation,
5) a cell and/or receptor inhibition,
6) an activation or inhibition of receptor and/or nanostructure internalization, and/or
7) a clustering of cells, preferably a clustering of cells inducing immune cell activation,
and/or wherein said desired spatial organization is
8) an epitope-to-cell-surface distance, e.g. a receptor height, and/or
9) an epitope-to-epitope distance, e.g. a receptor proximity.

13. The method according to claim 12, wherein said nanostructure further comprises at least one dye molecule and/or wherein said nanostructure is a nanostructure of any one of claims 1 to 4.

14. The method according to claim 12 or 13, wherein said qualitatively or quantitatively determining a response in step c) is performed using any of fluorescence measurements, e.g. flow cytometry, fluorescence microscopy, or via microplate reader, luminescence measurements, microscopy, a colorimetric measurement, and/or cell-surface marker staining.

15. A method of producing a bi- or multispecific targeting agent-comprising molecule, preferably a bi- or multispecific antibody or antigen-binding fragment thereof, comprising the steps:
i) performing the method of identifying a targeting agent combination, as defined in any one of claims 12-14, and
ii) producing a bi- or multispecific targeting agent-comprising molecule, preferably a bi- or multispecific antibody or antigen-binding fragment thereof, having the targeting agent combination identified in step d) of the method of identifying a targeting agent combination.

16. A method of screening a sample for a target having at least two target molecules, comprising the steps:
1) providing a nucleic acid nanostructure comprising a targeting agent combination of at least two targeting agents,
wherein, optionally, said nanostructure further comprises at least one dye molecule and/or said nanostructure is a nanostructure of any one of claims 1 to 4, 2)
contacting said nanostructure with a sample to be tested for the target having at least two target molecules,
3) qualitatively or quantitatively determining the target in said sample.
